# EUROPEAN PATENT SPECIFICATION

(11) **EP 2 013 221 B1**
(45) Date of publication and mention of the grant of the patent: **24.06.2015**
(21) Application number: 06769952.0
(22) Date of filing: 04.05.2006
(51) Int. Cl.: C07F 13/00

(54) **COMPOSITIONS AND METHODS FOR CELLULAR IMAGING AND THERAPY**
ZUSAMMENSETZUNGEN UND VERFAHREN ZUR ZELLULÄREN BILDDARSTELLUNG UND THERAPIE
COMPOSITIONS ET MÉTHODES POUR IMAGERIE CELLULAIRE ET THÉRAPIE

(30) Priority: 19.04.2006 US 745148 P
(43) Date of publication of application: 14.01.2009
(73) Proprietor: The Board of Regents of The University of Texas System, Austin, TX 78702-2981 (US)
(72) Inventor: YANG, David, J., Sugar Land, TX 77478 (US); OH, Chang, Sok, Houston, TX 77077 (US); YU, Dong-Fang, Houston, TX 77030 (US); AZHDARINIA, Ali, Houston TX 77077 (US); KOHANIM, Saady, Sugar Land, TX 77479 (US)
(74) Representative: Dehmel, Albrecht
(86) International application number: PCT/US2006/016784
(87) International publication number: WO 2007/120153

(56) References cited:
- WO-A2-2004/062574
- US-A- 4 988 496
- US-A1- 2003 053 954
- LI ET AL: "A Calcium-Sensitive Magnetic Resonance Imaging Contrast Agent" JOURNAL OF THE AMERICAN CHEMICAL SOCIETY, AMERICAN CHEMICAL SOCIETY LNKD- DOI:10.1021/JA983702L, vol. 121, no. 6, 1 January 1999 (1999-01-01), XP002115324 ISSN: 0002-7863
- DAVID J YANG ET AL: "Targeting EGFR-TK with radiolabeled cyclam-tyrosine", AMERICAN ASSOCIATION FOR CANCER RESEARCH. PROCEEDINGS OF THE ANNUAL MEETING; 97TH ANNUAL MEETING OF THE AMERICAN-ASSOCIATION-FOR-CANCER-RESEARCH (AACR); WASHINGTON, DC, USA; APRIL 01 -05, 2006, AMERICAN ASSOCIATION FOR CANCER RESEARCH, US; BIOSCIENCE, vol. 47, 1 April 2006 (2006-04-01), page 2394, XP002691881, ISSN: 0197-016X

## Description

### BACKGROUND OF THE INVENTION

### 1. Field of the Invention

The present invention relates generally to the fields of chemistry and radionuclide imaging. More particularly, it concerns compositions and methods involving N4 compounds and derivatives.

### 2. Description of Related Art

Radionuclide imaging modalities (Positron Emission Tomography, PET; Single Photon Emission Computed Tomography, SPECT) map the location and concentration of radionuclide-labeled compounds. To improve the diagnosis, prognosis, planning and monitoring of tissue specific disease treatment, characterization of disease tissue is extensively determined by development of more disease specific pharmaceuticals. PET ¹⁸F-fluorodeoxyglucose (FDG) has been used to diagnose and evaluate tumors, myocardial infarctions, and neurological diseases. Although tumor metabolic imaging using ¹⁸F-FDG has been studied in the last two decades, its clinical practice is still limited by the factors such as easy access, availability and isotope cost. In addition, ¹⁸F chemistry is complex and requires longer synthesis times (e.g. ¹⁸F-FDG, 40 min-75 min), and it is difficult to produce multiple agents simultaneously. Thus, it would be desirable to develop a simple chelation technique for labeling agents using metallic isotopes for tissue specific targeted radioimaging and radiotherapy.

Improvement of scintigraphic tumor imaging will benefit from the development of more tumor specific radiopharmaceuticals. Due to greater tumor specificity, radiolabeled ligands as well as radiolabeled antibodies have opened a new era in scintigraphic detection of tumors and undergone extensive preclinical development and evaluation (Mathias *et al*., 1996, 1997a, 1997b). Radionuclide imaging modalities (e.g., PET, SPECT) are diagnostic cross-sectional imaging techniques that map the location and concentration of radionuclide-labeled radiotracers. Although CT and MRI provide considerable anatomic information about the location and the extent of tumors, these imaging modalities typically cannot adequately differentiate invasive lesions from edema, radiation necrosis, grading or gliosis. PET and SPECT can be used to localize and characterize tumors by measuring metabolic activity. Thus methods that allow for more specific imaging of tumors is desirable.

One approach for producing novel compounds for imaging has involved the use of ethylenedicysteine (EC) derivatives, which are distinct from the compositions of the present invention. Several compounds have been labeled with ^{99m}Tc using nitrogen and sulfur chelates (Blondeau *et al*., 1967; Davison *et al*., 1980). Bis-aminoethanethiol tetradentate ligands, also called diaminodithol compounds, are known to form very stable Tc(V)O complexes on the basis of efficient binding of the oxotechnetium group to two thiolsulfur and two amine nitrogen atoms. Radiometal complexes of 2-pyrrolthiones labeled with ^{99m}Tc-2-pyrrolthiones complexes have been developed for use as radiopharmaceuticals for imaging and therapy (WO 0180906A2). ^{99m}Tc-L,L-ethylenedicysteine (^{99m}Tc-EC) is a recent and successful example of N₂S₂ chelates. EC can be labeled with ^{99m}Tc easily and efficiently with high radiochemical purity and stability, and is excreted through the kidney by active tubular transport *(Surma et al*., 1994; Van Nerom *et al*., 1990, 1993; Verbruggen *et al*., 1990, 1992). Furthermore, ^{99m}Tc chelated with ethylenedicysteine (EC) and conjugated with a variety of ligands has been developed for use as an imaging agent for tissue-specific diseases, as a prognostic tool, and as a tool to deliver therapeutics to specific sites within a mammalian body (WO 0191807A2, AU 0175210A5). ^{99m}Tc-EC-chelates have been developed for renal imaging and examination of renal function (U.S. Patent 5,986,074 and U.S. Patent 5,955,053). A method of preparing ^{99m}Tc-EC complexes and a kit for performing said method has also been developed (U.S. Patent 5,268,163 and WO 9116076A1). U.S. Patent 6,691,724 discloses ethylenecysteine drug conjugates.

The compound cyclam-tyrosine, as well as metal complexes thereof and their use in imaging, were presented by David J Yang et al.: "Targeting EGFR-TK with radiolabeled cyclam-tyrosine" (American Association for Cancer Research. Proceedings of the Annual Meeting; 97th Annual Meeting of the American Association for Cancer Research (AACR); Washington, DC, USA; April 01 -05, 2006; Bioscience, vol. 47, 1 April 2006 (2006-04-01), page 2394).

### SUMMARY OF THE INVENTION

The present invention presents compounds and methods relating to a simple chelation technique for labeling agents using metallic isotopes that may be used for tissue specific targeted radioimaging and radiotherapy.

An aspect of the present invention is directed towards a compound having the formula : 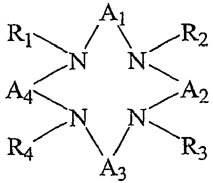 wherein A₁, A₂, A₃, and A₄, are each independently -(CH₂)_{X}-, wherein X=2-4; and R₁, R₂, and R₃ are each independently chosen from the group consisting of: hydrogen or 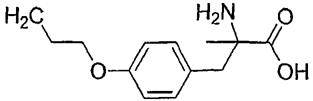 wherein R₄ is

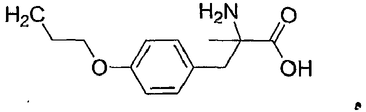

The compound may be chelated to a metal atom. Said metal atom may not be radioactive. Said metal atom may be copper, cobalt, platinum, iron, arsenic, rhenium, or germanium. Said compound may be chelated to a radionuclide. Said radionuclide may be ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu. In certain embodiment, said radionuclide is chosen from the group consisting of ⁶⁸Ga, ⁹⁰Y, ^{99m}Tc, ⁶⁸Ga, or ¹⁸⁸Re. Said radionuclide may be ^{99m}Tc. The compound may be comprised in a pharmaceutical composition.

Another aspect of the present invention relates to said compound for use in a method for the treatment of cancer. The subject may be a mammal, such as a human. The compound may be chelated to ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu. The compound may be administered in combination with a second anti-cancer compound, a radiation therapy, or surgery.

Another aspect of the present invention relates to said compound for use in a method for imaging. The subject may be a mammal, such as a human. The compound may be chelated to ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu. Said imaging may comprise PET imaging or SPET imaging.

Another aspect of the present invention relates to a kit comprising a compound of the present invention and a reducing agent. The kit may comprise a radionuclide. The radionuclide may be ^{99*m*}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu. The kit may comprise an antioxidant (e.g., vitamin C, tocopherol, pyridoxine, thiamine, or rutin). The kit may comprise a transition chelator (e.g., glucoheptonate, gluconate, glucarate, citrate, or tartarate). The reducing agent may be tin (II) chloride or triphenylphosphine.

The use of the word "a" or "an" when used in conjunction with the term "comprising" in the claims and/or the specification may mean "one," but it is also consistent with the meaning of "one or more," "at least one," and "one or more than one." As used herein "another" may mean at least a second or more.

It is contemplated that any embodiment discussed in this specification can be implemented with respect to any method or composition of the invention, and vice versa. Furthermore, compositions of the invention can be used to achieve the methods of the invention.

Throughout this application, the term "about" is used to indicate that a value includes the inherent variation of error for the device, the method being employed to determine the value, or the variation that exists among the study subjects.

The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps.

### BRIEF DESCRIPTION OF THE DRAWINGS

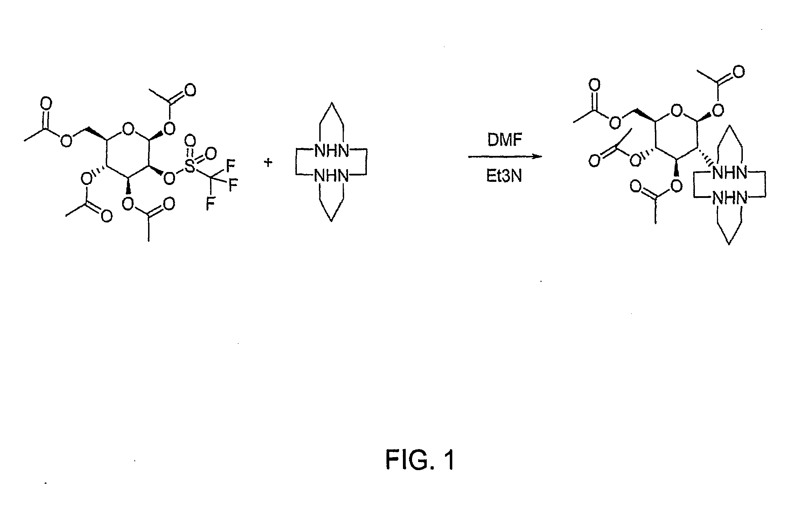

**FIG. 1****.** Synthesis of N4-DG.

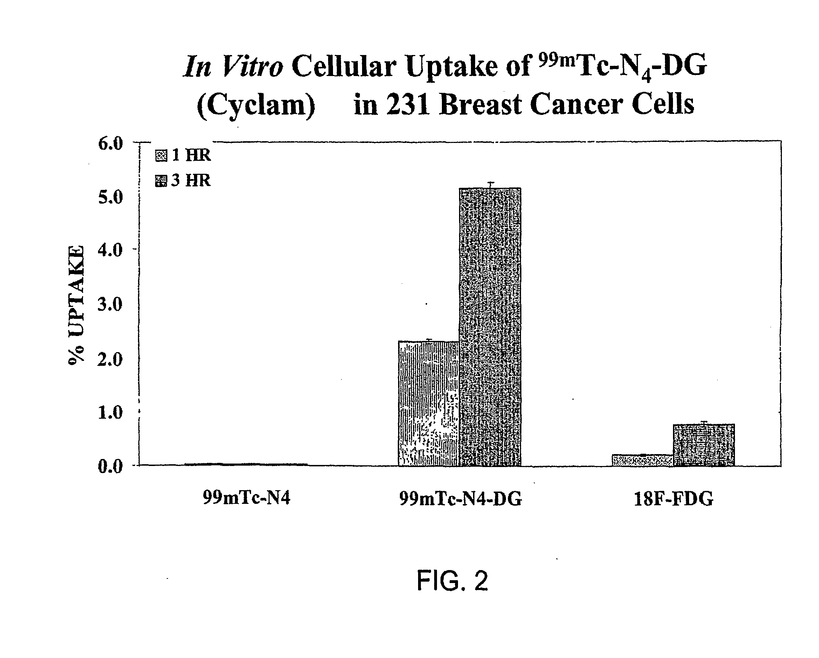

**FIG. 2****.** *In vitro* cellular uptake of ^{99m}Tc-N4-DG (Cyclam) in 231 breast cancer cells. 50,000 cells/well were plated and allowed to reach 70-80% confluency. Tracers were administered at 4 µCi/well and incubated at 37°C for 1-3 hrs. Cells were then harvested and radioactivity was counted and quantified.

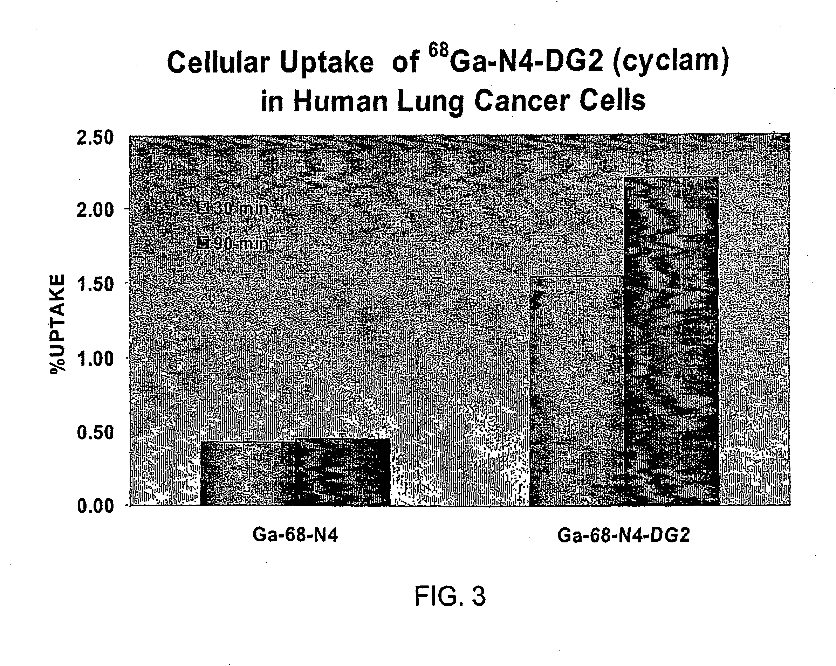

**FIG. 3****.** Cellular uptake of ⁶⁸Ga-N4-DG2 (cyclam) in human lung cancer cells. *In vitro* cellular uptake using A549 cells showed increased uptake of ⁶⁸Ga-N4-DG whereas ⁶⁸Ga-N4 showed poor uptake.

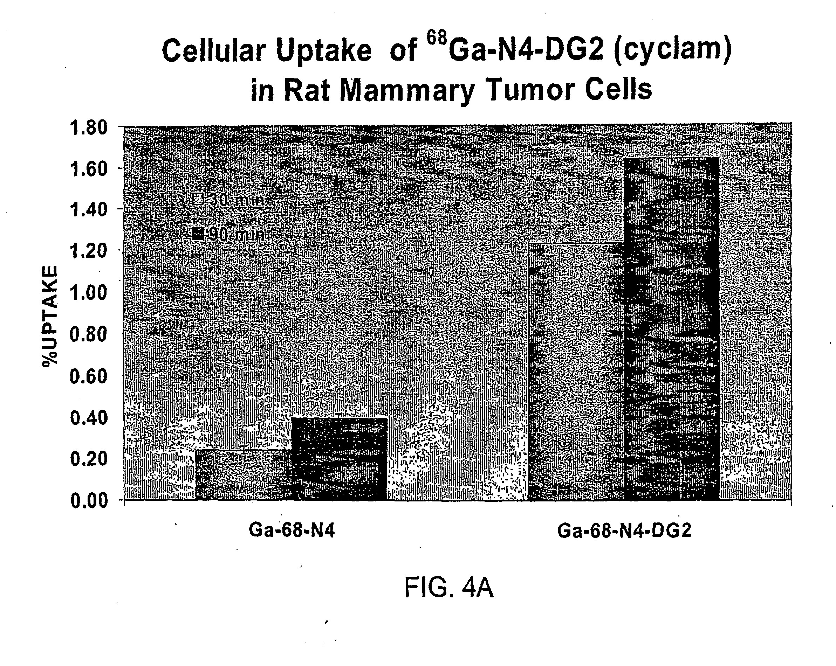

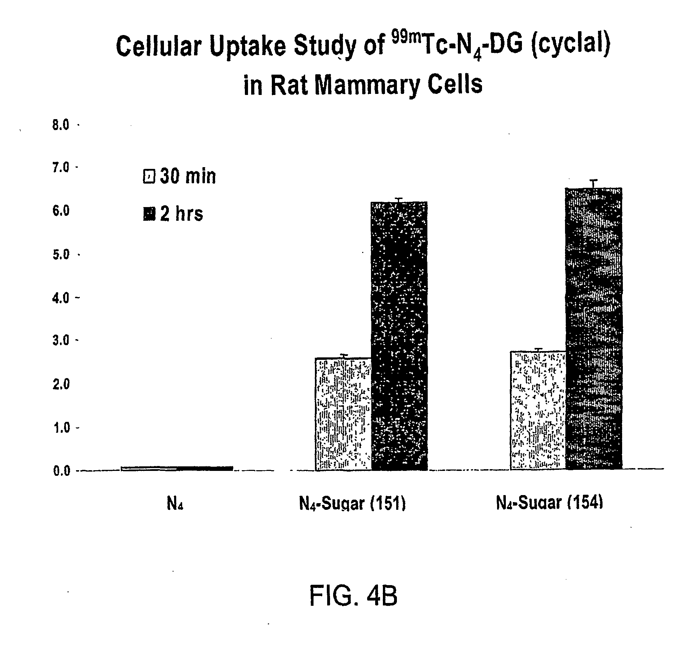

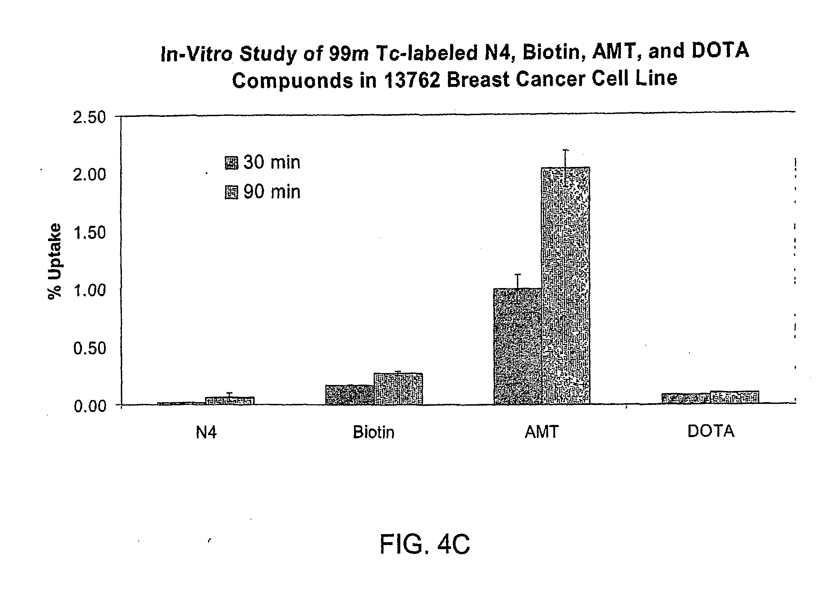

**FIGS. 4A-C****.** **FIG. 4A****,** Cellular uptake of ⁶⁸Ga-N4-DG2 (cyclam) in rat mammary tumor cells. *In vitro* cellular uptake using 13762 cells showed increased uptake of ⁶⁸Ga-N4-DG whereas ⁶⁸Ga-N4 showed poor uptake. **FIG. 4B****,** Cellular uptake study of ^{99m}Tc-N4-DG (cyclal). 50,000 cells/well were plated and allowed to reach 70-80% confluency. Tracers were administered at 4 µCi/well and incubated at 37°C for 0.5-2 hrs. Cells were then harvested and radioactivity was counted and quantified. **FIG. 4C****,** *In vitro* study of ^{99m}Tc-labeled N4, Biotin, AMT, and DOTA compuonds in 13762 breast cancer cell line. 50,000 cells/well were plated and allowed to reach 70-80% confluency. Tracers were administered at 4 µCi/well and incubated at 37°C for 0.5-1.5 hrs.

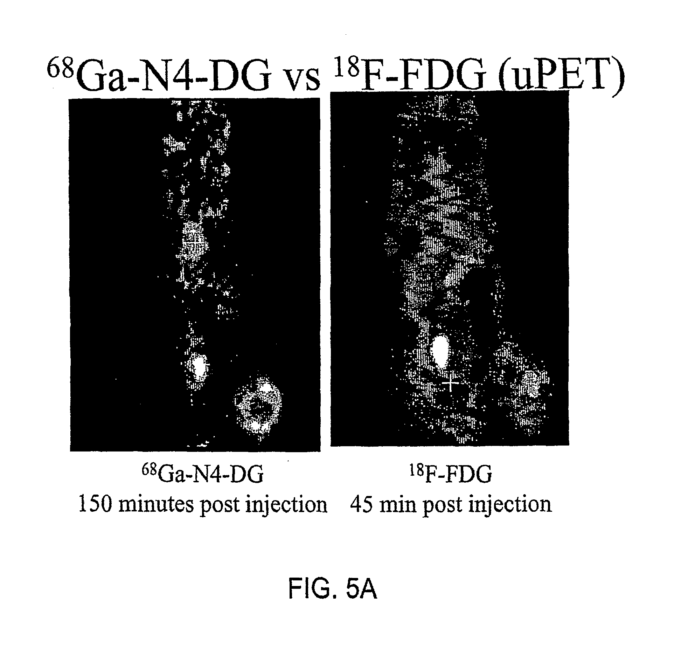

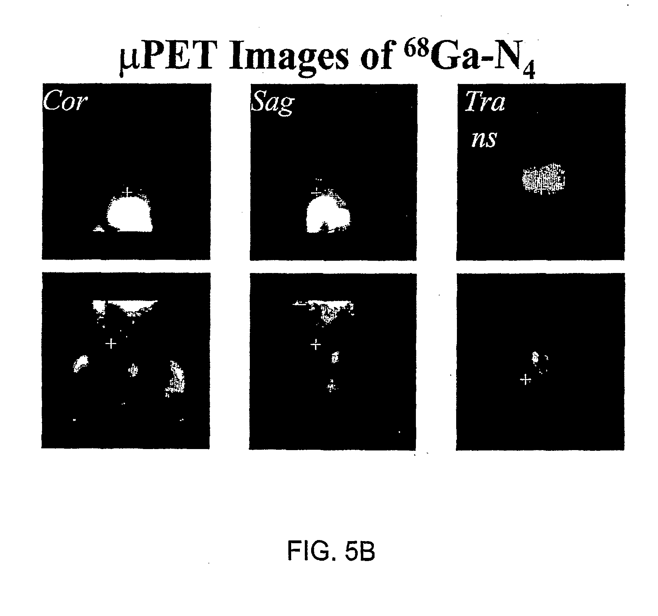

**FIGS. 5A-B.** **FIG**. **5A****,** ⁶⁸Ga-N4-DG vs ¹⁸F-FDG (uPET). A similar distribution pattern was observed between ⁶⁸Ga-N4-DG (cyclam) and ¹⁸F-FDG. **FIG. 5B****,** µPET Images of ⁶⁸Ga-N4. Mammary tumor-bearing rats injected with 400 µCi ⁶⁸Ga-N4. Selected images were shown at 2 hours post-injection.

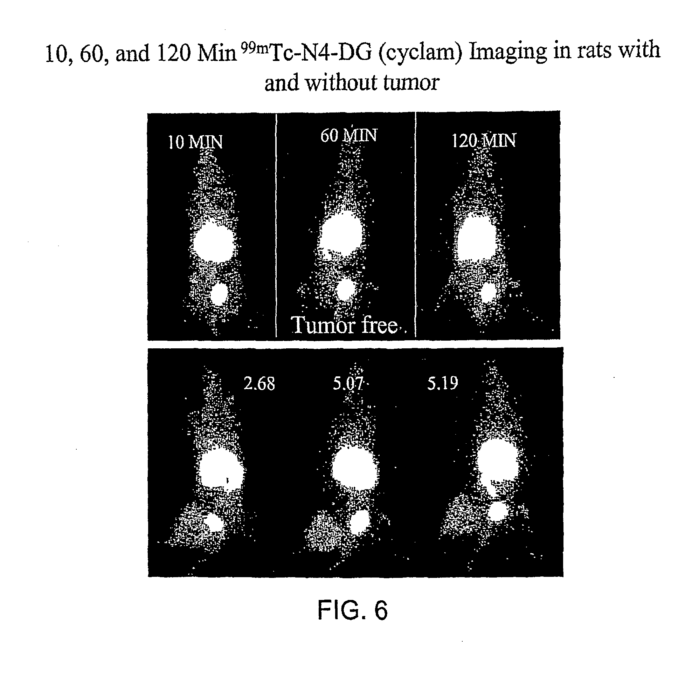

**FIG. 6****.** 10, 60, and 120 Min ^{99m}Tc-N4-DG (cyclam) Imaging in rats with and without tumor. 10, 60, and 120 min Planar scintigraphy of ^{99m}Tc-N4-DG in rats with and without tumor (breast tumor cell line) after 300 mCi /rat, i.v. injection, acquired 500,000 count to demonstrate tumor visualization. Tumor-to-non tumor ratios are shown. T=tumor and M=muscle.

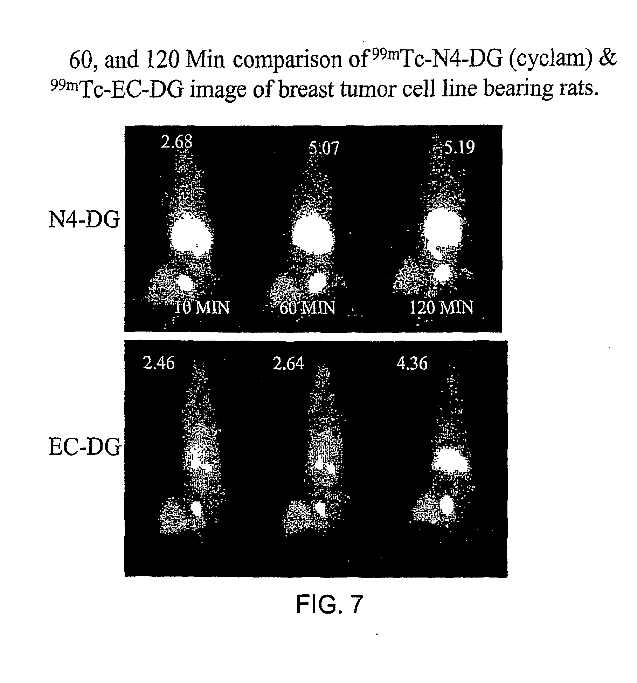

**FIG 7****.** 10, 60, and 120 min comparison of ^{99m}Tc-N4-DG (cyclam) & ^{99m}Tc-EC-DG image of breast tumor cell line bearing rats. 60, and 120 min Planar scintigraphy of ^{99m}Tc-N4-DG & ^{99m}Tc-EC-DG comparison in breast tumor cell line bearing rats. (300 mCi /rat, i.v. injection, acquired 500,000 count). Tumor-to-non tumor ratios are shown. T=tumor and M=muscle.

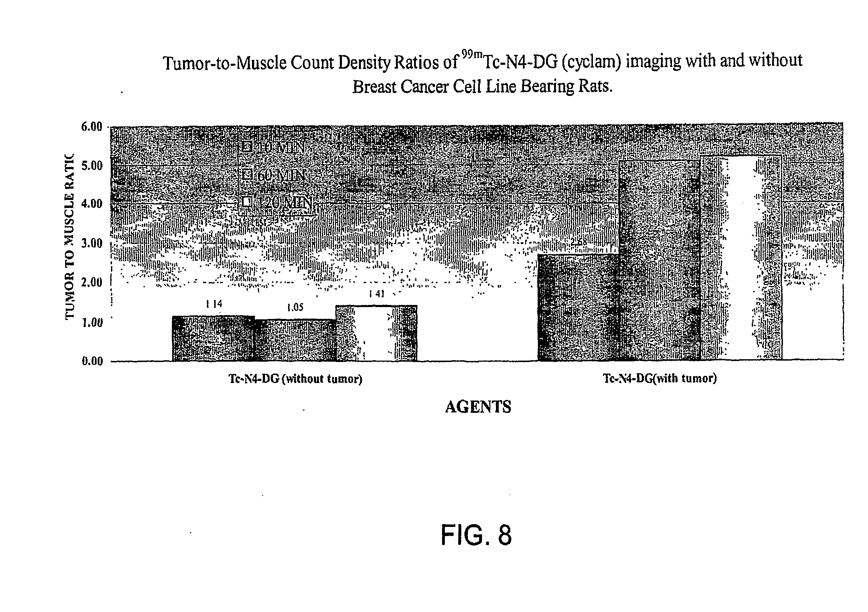

**FIG 8****.** Tumor-to-muscle count density ratios of ^{99m}Tc-N4-DG (cyclam) imaging with and without breast cancer cell line bearing rats. Increased tumor-to-muscle ratios was observed with ^{99m}Tc-N4-DG.

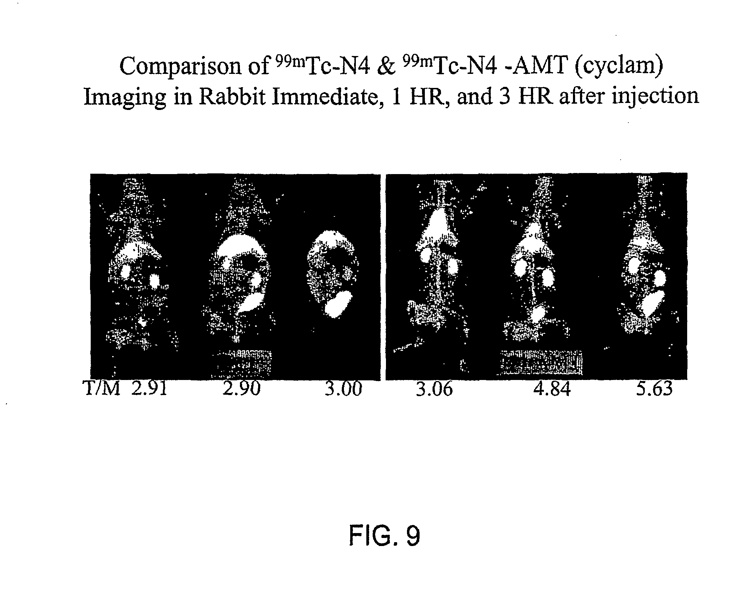

**FIG. 9****.** Comparison of ^{99m}Tc-N4 & ^{99m}Tc-N4 -AMT (cyclam) imaging in rabbit immediate, 1 hr, and 3 hr after injection. Planar scintigraphy of ^{99m}Tc-N4 & ^{99m}Tc-N4-AMT in VX2 tumor-bearing rabbits (1 mCi/rabbit, i.v. injection) to compare tumor visualization. Increased tumor/muscle ratios were seen in ^{99m}Tc-N4-AMT groups.

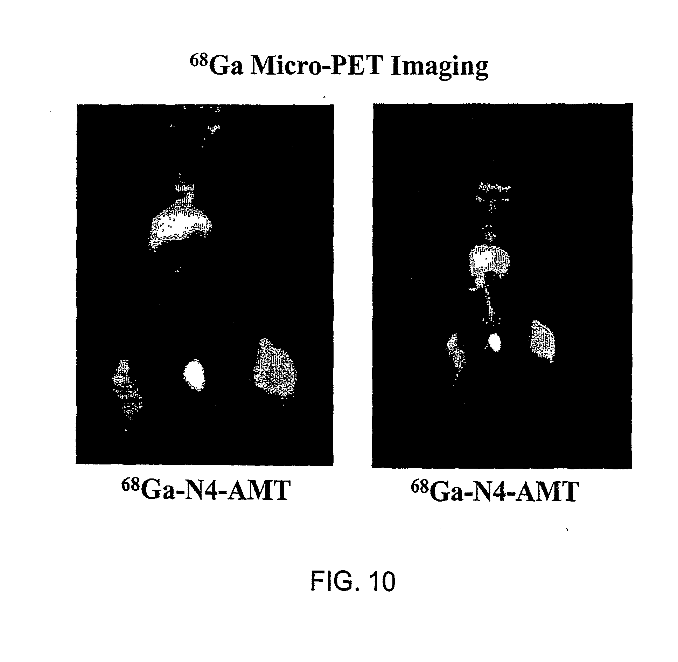

**FIG. 10****.** ⁶⁸Ga micro-PET imaging. Mammary tumor-bearing rats injected with 400 µCi ⁶⁸Ga-N4-AMT. Whole body images showed that tumor (right leg) could be imaged at 2 hours post-injection.

### DESCRIPTION OF ILLUSTRATIVE EMBODIMENTS

In the field of nuclear medicine, certain pathological conditions are localized, or their extent is assessed, by detecting the distribution of small quantities of internally-administered radioactively labeled tracer compounds (called radiotracers or radiopharmaceuticals). Methods for detecting these radiopharmaceuticals are known generally as imaging or radioimaging methods.

### I. DEFINITIONS

The word "conjugate" and "conjugated" is defined herein as chemically joining within the same molecule. For example, two or more molecules and/or atoms may be conjugated together via a covalent bond, forming a single molecule. The two molecules may be conjugated to each other via a direct connection (e.g., where the compounds are directly attached via a covalent bond) or the compounds may be conjugared via an indirect connection (e.g., where the two compounds are covalently bonded to one or more linkers, forming a single molecule). In other instances, a metal atom may be conjugated to a molecule via a chelation interaction.

The term "N4 derivative" is defined herein as an N4 compound that has been conjugated to at least one other molecule or atom. In certain embodiments the derivative comprises a N4 compound that has an atom chelated to it. The N4 derivative may comprise a N4 compound that is conjugated to a targeting ligand (*e.g.*, via a covalent bond) and/or a linker *(e.g.,* via a covalent bond) and/or a metal chelate *(e.g.,* via a chelation interaction).

As used herein the term "radionuclide" is defined as a radioactive nuclide (a species of atom able to exist for a measurable lifetime and distinguished by its charge, mass, number, and quantum state of the nucleus) which, in specific embodiments, disintegrates with emission of corpuscular or electromagnetic radiation. The term may be used interchangeably with the term "radioisotope".

The term "therapeutic agent" as used herein is defined as an agent which provides treatment for a disease or medical condition. The agent in a specific embodiment improves at least one symptom or parameter of the disease or medical condition. For instance, in tumor therapy, the therapeutic agent reduces the size of the tumor, inhibits or prevents growth or metastases of the tumor, or eliminates the tumor. Examples include a drug, such as an anticancer drug, a gene therapy composition, a radionuclide, a hormone, a nutriceutical, or a combination thereof.

The term "tumor" as used herein is defined as an uncontrolled and progressive growth of cells in a tissue. A skilled artisan is aware other synonymous terms exist, such as neoplasm or malignancy. In a specific embodiment, the tumor is a solid tumor. In other specific embodiments, the tumor derives, either primarily or as a metastatic form, from cancers such as of the liver, prostate, pancreas, head and neck, breast, brain, colon, adenoid, oral, skin, lung, testes, ovaries, cervix, endometrium, bladder, stomach, and epithelium.

The term "drug" as used herein is defined as a compound which aids in the treatment of disease or medical condition or which controls or improves any physiological or pathological condition associated with the disease or medical condition.

The term "anticancer drug" as used herein is defined as a drug for the treatment of cancer, such as for a solid tumor. The anticancer drug preferably reduces the size of the tumor, inhibits or prevents growth or metastases of the tumor, and/or eliminates the tumor. The terms "anticancer drug", "anti-cancer drug", and "anti-cancer compound" are used interchangeably herein.

As used herein the specification, "a" or "an" may mean one or more. As used herein in the claim(s), when used in conjunction with the word "comprising", the words "a" or "an" may mean one or more than one. As used herein "another" may mean at least a second or more.

### II. N4 COMPOUNDS AND DERIVATIVES

The present invention provides a method by which N4 compounds, which are typically hydrophobic chelators, may be conjugated to hydrophobic molecules to produce novel compounds which may be used for purposes including imaging and radiotherapy. Certain N4 compounds may be obtained from commercial source such as Sigma chemical company (St. Louis, MO) and Aldrich Chemical company (Milwaukee, WI). U.S. Patent 5,880,281 describes a method for producing certain N4 compounds.

N4 compounds are defined herein as having the structure: 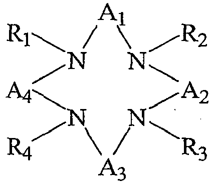 wherein A₁, A₂, A₃, and A₄ are alkyl; and
wherein R₁, R₂, R₃, and R₄ are hydrogen. Several N4 compounds are shown below.

### Structures of N4-compounds

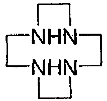

**1) Registry Number:** 294-90-6
   **CA Index Name:** 1,4,7,10-Tetraazacyclododecane (6CI,8CI,9CI)
   **Other Names:** Cyclen; NSC 629374; Tetraaza-12-crown-4 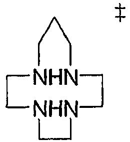
**2) Registry Number:** 295-14-7
   **CA Index Name:** 1,4,7,10-Tetraazacyclotridecane (6CI,8CI,9CI)
   **Other Names:** Cyclam 13 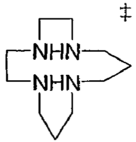
**3) Registry Number:** 52877-36-8
   **CA Index Name:** 1,4,7,11-Tetraazacyclotetradecane (9CI)
   **Other Names:** Isocyclam 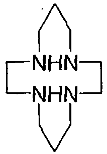
**4) Registry Number:** 295-37-4
   **CA Index Name:** 1,4,8,11-Tetraazacyclotetradecane (6CI,7CI,8CI,9CI)
   **Other Names:** Cyclam; JM 1498; NSC 180811 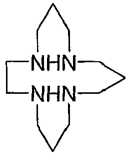
**5) Registry Number:** 15439-16-4
   **CA Index Name:** 1,4,8,12-Tetraazacyclopentadecane (8CI,9CI)
   **Other Names:** Cyclal 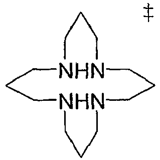
**6) Registry Number:** 24772-41-6
   **CA Index Name:** 1,5,9,13-Tetraazacyclohexadecane (8CI,9CI) 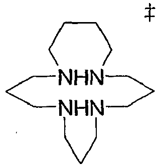
**7) Registry Number:** 43031-32-9
   **CA Index Name:** 1,5,9,13-Tetraazacycloheptadecane (9CI) 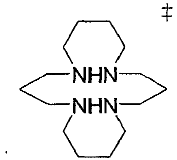
**8) Registry Number:** 68966-28-9
   **CA Index Name:** 1,5,10,14-Tetraazacyclooctadecane (9CI) 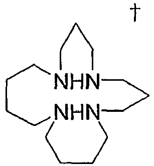
**9) Not existing compound**
   **Name:** 1,5,9,14-Tetraazacyclooctadecane 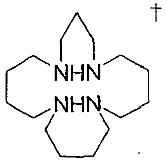
**10) Not existing compound**
   **Name:** 1,5,10,15-Tetraazacyclononadecane 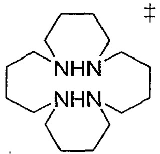
**11) Registry Number:** 3713-77-7
   **CA Index Name:** 1,6,11,16-Tetraazacycloeicosane (8CI,9CI)
   ^{†} : Not existing compound
   ^{‡} : Not commercially available

N4 compounds can be used as chelators. For example, cyclam and other N4 compounds were tested for their ability to alleviate acute cadmium poisoning (Srivastava *et al*., 1996). U.S. Patent 4,141,654 describes certain compounds with structural similarity to N4 compounds that may be used to chelate actiriide ions. U.S. Patent 5,648,063 discloses compounds with structural similarity to N4 compounds which can chelate metal ions and may also be used in certain NMR diagnostic procedures. U.S. Patent 6,071,490 utilizes a modified cyclen for PET imaging. U.S. Patent 6,613,305 discloses Vitamin B₁₂ attached to various N4 compounds.

### A. Targeting ligands

In the present specification, it is generally preferable to conjugate a targeting moiety (e.g., a hydrophobic anticancer drug) to the N4 compound; however, in certain embodiments a N4 compound that is not conjugated to a targeting moiety may be used for imaging and therapy. A targeting moiety may be conjugated to the N4 compound via several methods. One method is to synthesize a halide (e.g., iodinated) targeting moiety. For example, the hydroxy group of a targeting moiety *(e.g.,* a hydrophobic molecule) may be converted to a tosyl-, mesyl-, triflate or halide *(e.g.,* iodine) group. The reaction condition is typically performed in an organic solvent (dimethylformamide, DMF). In certain embodiments, the final product is soluble in water after hydrochloride salt formation. Alternatively, another method to conjugate N4 compound to a targeting moiety is to synthesize a sulfonate *(e.g.,* tosyl- mesyl or triflate) targeting moiety. Di-, tri- or all substitutes on the N4 compound may be prepared by reacting these iodinated or sulfonate targeting agents. For mono-substitutes, a selective protection of nitrogen groups is needed. Targeting ligands that may be conjugated with an N4 compound include amino acids *(e.g.,* tyrosine, serine), amino acid derivaitves *(e.g.,* alphamethyltyrosine), tamoxifen, estrone, and tetraacetate mannose.

Other ligands may also be conjugated to the N4 compound. In general, the ligands will possess either a halide or a hydroxy group that are able to react with and covalently bind to the N4 compound on either one or both acid arms. Ligands contemplated include, but are not limited to, angiogenesis/antiangiogenesis ligands, DNA topoisomerase inhibitors, glycolysis markers, antimetabolite ligands, apoptosis/hypoxia ligands, DNA intercalators, receptor markers, peptides, nucleotides, antimicrobials such as antibiotics or antifungals, organ specific ligands and sugars, and agents that mimic glucose.

It is contemplated that virtually any targeting ligand that is known, or may be subsequently discovered, that possesses a hydroxy group or a halide, or alternatively may have a hydroxy group or halide introduced into its structure *(e.g.,* via the addition of a sidechain, or by attaching a halide to a phenol group in the targeting ligand), may be used . In certain embodiments, a targeting ligand may be directly conjugated to a N4 compound *(e.g.,* via a covalent bond between the targeting ligand and the N4 compound), or a targeting ligand may be indirectly conjugated to a N4 compound via a linker. It is envisioned that targeting ligands that have previously been conjugated to another (non-N4 compound) chelator, such as EC, may be conjugated to N4 compounds and used for therapeutic purposes; in certain instances, it may be required to modify the targeting ligand *(e.g.,* adding a side chain that contains a hydroxyl or a halide) in order to covalently bind the targeting ligand to the N4 compound. For example, covalent binding of ligands with EC compounds are typically performed in water, and in certain instances it may be preferred to covalently attach a targeting ligand with a N4 compound by utilizing a reaction in an organic solvent; in these instances, a targeting ligand that can be covalently bound to EC via a reaction in water may be modified *(e.g.,* a halide or hydroxy group may be introduced to the structure) to allow the targeting ligand to be covalently bound to a N4 compound via a reaction in an organic solvent.

N4 derivatives may be used to target tumors *(e.g.,* cancerous, precancerous, benign), tumor angiogenesis, hypoxia, apoptosis defects, disease receptors *(e.g.,* cell receptors that are indicative of cancer), disease functional pathways *(e.g.,* a metabolic pathway that has been altered by a disease state), and disease cell cycles. Additionally, N4 derivatives may be used for the assessment of a pharmaceutical agent's effectiveness on these biochemical processes.

N4 derivatives may also be used as a diagnostic tool and/or for predicting responses to certain kinds of treatment. For example, an N4 derivative that comprises tamoxifen (an estrogen receptor targeting ligand) may be used to image cancerous tumors; in this example, the imaging may provide important information about the disease such as to what degree the cancerous cells express the estrogen receptor which can be used to predict how the disease will respond to treatments that target cells expressing the estrogen receptor *(e.g.,* when it is identified that cancerous tumors selectively express high levels of estrogen receptor, this information indicates that the cancerous cells will likely respond to therapeutic doses of anti-cancer agents that target cells expressing the estrogen receptor). This approach is referred to as "image guided therapy".

An advantage of conjugating a N4 compound with a tissue targeting ligands is that the specific binding properties of the tissue targeting ligand can concentrate the radioactive signal over the area of interest. It is envisioned that N4 derivatives used for imaging and/or therapy may comprise a N4 compound conjugated to a targeting ligands designed for targeting cancerous tumors, pre-cancerous tumors, disease receptors, hypoxic tissues (hypoxia), apoptosis pathways, disease cell cycles, and/or disease functional pathways. N4 derivatives may also be used for assessing a pharmaceutical agent's effectiveness on various metabolic and/or biochemical pathways or individual reactions. Examples of certain targeting ligands which may be used the present invention can be found in Table 1. In certain embodiments, an anti-cancer drug may be used as a targeting ligand. Anti-cancer drugs are well known in the art *(e.g.,* Connors,1996). Table 2 in U.S. Patent 6,691,724 lists several examples of anti-cancer drugs which may be used as targeting ligands.

**Table 1**

| **Targets for N4 Derivatives** | **Examples of Targeting Ligands** |
|---|---|
| Tumor Angiogenesis | Celecoxib, C225, angiostatin |
| Disease Receptor | tamoxifen, α-β tyrosine, tyrosine, alpha methyltyrosine, luteinizing hormone, transferrin, somatostatin, androgen, estrogen, estrone, progesterone, tetraacetate mannose, |
| Disease Cell Cycle | adenosine, penciclovir |
| Pharmaceutical Agent Assessment | carnitine, puromycin |
| Apoptosis Targeting | TRAIL monoclonal antibody, caspase-3 substrate, Bcl family member |

### 1. Tumor Angiogenesis Targeting

Throughout this application, "tumor angiogenesis targeting" refers to the use of an agent to bind to tumor neovascularization and tumor cells. Agents that are used for this purpose are known to those of ordinary skill in the art for use in performing various tumor measurements, including measurement of the size of a tumor vascular bed, and measurement of tumor volume. Some of these agents bind to the vascular wall. One of ordinary skill in the art would be familiar with the agents that are available for use for this purpose. A tumor angiogenesis targeting ligand is a ligand that is used for the purpose of tumor angiogenesis targeting as defined above. Examples of tumor angiogenesis targeting ligands include celecoxib, C225, herceptin, angiostatin, and thalidomide, which have been developed for the assessment of biochemical process on angiogenesis.

In certain embodiments, a tumor targeting ligand may associate with tumor tissues by targeting the hypoxia associated with tumor cells. Examples of tumor targeting ligands that target hypoxic tissues include nitroimidazole and metronidazole, and these ligands may also be used to target other hypoxic tissues that are hypoxic due to a reason other than cancer (e.g., stroke).

### 2. Tumor Apoptosis Targeting

"Tumor apoptosis targeting" refers to the use of an agent to bind to a cell that is undergoing apoptosis or is at risk of undergoing apoptosis. These agents are generally used to provide an indicator of the extent or risk of apoptosis, or programmed cell death, in a population of cells, such as a tumor. One of ordinary skill in the art would be familiar with agents that are used for this purpose. Certain examples of apoptosis targeting agents are shown in Table 1. A "tumor apoptosis targeting ligand" is a ligand that is capable of performing "tumor apoptosis targeting" as defined in this paragraph. Examples of a tumor apoptosis ligand includes a TRAIL (TNF-related apoptosis inducing ligand) monoclonal antibody. TRAIL is a member of the tumor necrosis factor ligand family that rapidly induces apoptosis in a variety of transformed cell lines. Other examples of tumor apoptosis targeting ligands include a substrate of caspase-3, such as peptide or polypeptide that includes the 4 amino acid sequence aspartic acid-glutamic acid-valine-aspartic acid.

Significant research is directed towards the creation and evaluation of new compounds that affect apoptosis, such as restoring apoptosis sensitivity to cancer cells (Reed, 2003). It is envisioned that the present invention may be used to expedite the evaluation and/or efficacy of known and/or subsequently discovered tumor apoptosis targeting compounds.

### 3. Disease Receptor Targeting

In "disease receptor targeting," certain agents are exploited for their ability to bind to certain cellular receptors that are overexpressed in disease states, such as cancer. Examples of such receptors which are targeted include estrogen receptors, amino acid transporters, androgen receptors, pituitary receptors, transferrin receptors, progesterone receptors, and glucose transporters. Examples of agents that can be applied in disease-receptor targeting are shown in Table 1. Disease receptor targeting ligands (*e.g*., pentetreotide, octreotide, transferrin, and pituitary peptide) bind to cell receptors, some of which are overexpressed on certain cells.

Estrogen, estrone, and tamoxifen target the estrogen receptor. Estrogen receptors are over expressed in certain kinds of cancer, and N4 derivatives that comprise an estrogen receptor targeting ligand may be used in certain embodiments to image tumors. The expression of estrogen receptors is also altered in the diseases of osteoporosis and endometriosis. It is anticipated that an N4 derivative comprising an estrogen receptor targeting ligand may be used to image other diseases such as osteoporosis and endometriosis.

Glucose transporters are overexpressed in various diseased cells such as certain cancerous cells. Tetraacetate mannose, deoxyglucose, certain polysaccharides (e.g., neomycin, kanamycin, tobramycin), and monosaccharides (e.g., glucosamine) also bind the glucose transporter and may be used as disease receptor targeting ligands. Since these ligands are not immunogenic and are cleared quickly from the plasma, receptor imaging would seem to be more promising compared to antibody imaging.

Similarly, amino acid transporters are also overexpressed in various diseased cells such as certain cancerous cells. Amino acids and/or amino acid derivatives (e.g., serine, tyrosine, alpha methyltyrosine) may be used as disease receptor targeting ligands.

Additional receptor targeting ligands are available and may be conjugated to N4 compounds. Other examples of disease receptor targeting ligands include leuteinizing hormone and transferrin. Folic acid, folate, tomudex, and methotrexate are examples of disease receptor targeting ligands that bind folate receptors.

"Tumor targeting" refers to the ability of a compound to preferentially associate with tumors (*e.g*., cancerous, pre-cancerous, and/or benign). A "tumor targeting ligand" refers to a compound which preferentially binds to or associates with tumor tissues, as compared to non-tumor tissues. Ligands (e.g., small molecules or antibodies) which preferentially target tumors are well known in the art.

### 4. Disease Cell Cycle Targeting

Disease cell cycle targeting refers to the targeting of agents that are upregulated in proliferating cells. Compounds used for this purpose can be used to measure various parameters in cells, such as tumor cell DNA content.

Certain disease cell cycle targeting ligands are nucleoside analogues. For example, pyrimidine nucleoside (e.g., 2'-fluoro-2'-deoxy-5-iodo-1-ß-D-arabinofuranosyluracil [FIAU], 2'-fluoro-2'-deoxy-5-iodo-1-ß-D-ribofuranosyl-uracil [FIRU], 2'-fluoro-2'-5-methyl-1-ß-D-arabinofuranosyluracil [FMAU], 2'-fluoro-2'-deoxy-5-iodovinyl-1-ß-D-ribofuranosyluracil [IVFRU]) and acycloguanosine: 9-[(2-hydroxy-1-(hydroxymethyl)ethoxy)methyl]guanine (GCV) and 9-[4-hydroxy-3-(hydroxy-methyl)butyl]guanine (PCV) (Tjuvajev *et al*., 2002; Gambhir *et al*., 1998; Gambhir *et al*., 1999) and other ¹⁸F-labeled acycloguanosine analogs, such as 8-fluoro-9-[(2-hydroxy-1-(hydroxymethyl)ethoxy)methyl]guanine (FGCV) (Gambhir *et al*., 1999; Namavari *et al*., 2000), 8-fluoro-9-[4-hydroxy-3-(hydroxymethyl)butyl]guanine (FPCV) (Gambhir *et al*., 2000; Iyer *et al*., 2001), 9-[3-fluoro-1-hydroxy-2-propoxy methyl]guanine (FHPG) (Alauddin *et al*., 1996; Alauddin *et al*., 1999), and 9-[4-fluoro-3-(hydroxymethyl)butyl]guanine (FHBG) (Alauddin and Conti, 1998; Yaghoubi *et al*., 2001) have been developed as reporter substrates for imaging wild-type and mutant (Gambhir *et al*., 2000) HSV1-tk expression. One or ordinary skill in the art would be familiar with these and other agents that are used for disease cell cycle targeting.

Examples of disease targeting ligands include, for example, adenosine and penciclovir. The antiviral nucleoside analog FHBG (a penciclovir analog), another disease targeting ligand, has for *in vivo* measurement of cell proliferation using PET (Alauddin *et al*., 2001)

### 5. Disease for Hypoxia Targeting

Disease cell hypoxia targeting refers to the targeting of agents that are upregulated in hypoxic cells. Compounds used for this purpose can be used to measure various parameters in cells, such as tumor cell hypoxia, resistance or residual content.

Certain disease cell hypoxia targeting ligands include 2- or 5-nitroimidazole analogues. For example, misonidazole (2-nitroimidazole) and metronidazole (5-nitroimidazole) analogues.

### 6. Disease for Glycolysis Targeting

Disease cell glycolysis targeting refers to the targeting of agents that are upregulated by glucose utilization in cells. Compounds used for this purpose can be used to measure various parameters in cells, such as tumor cell growth, inflammation degrees. Disease cell glycolysis targeting ligands include glucose, galactose, mannose and ribose analogues.

### B. Linkers

If amino or hydroxy groups are not available (*e.g*., acid functional group), a desired ligand may still be conjugated to the N4 compound (which may or may not be radiolabeled) using the methods disclosed herein by adding a linker, such as ethylenediamine, amino propanol, diethylenetriamine, aspartic acid, polyaspartic acid, glutamic acid, polyglutamic acid, or lysine. For example, U.S. Patent 6,737,247 discloses several linkers which may be used . U.S. Patent 5,605,672 discloses several "preferred backbones" which may be used as linkers. In certain embodiments, an N4 compound may be conjugated to a linker, and the linker is conjugated to the targeting ligand. In other embodiments more than one linker may be used; for example, an N4 compound may be conjugated to a linker, and the linker is conjugated to a second linker, wherein the second linker is conjugated to the targeting ligand. In, certain embodiments, two, three, four, or more linkers that are conjugated together may be used to conjugate an N4 compound and targeting ligand. However, it is generally preferable to only use a single linker to conjugate a N4 compound and a targeting ligand.

### C. N4 derivatives

The term "N4 derivative" is defined herein as an N4 compound that has been conjugated to at least one other molecule or atom. In certain embodiments the derivative comprises a N4 compound that has an atom chelated to it. The N4 derivative may comprise a N4 compound that is conjugated to a targeting ligand (e.g., via a covalent bond) and/or a linker *(e.g.,* via a covalent bond) and/or a metal chelate (*e.g., via* a chelation interaction).

Certain embodiments disclosed in the present specification relate to N4 derivatives, methods for producing N4 derivatives, and uses of N4 derivatives. In certain embodiments, an N4 derivative is a compound having the formula: 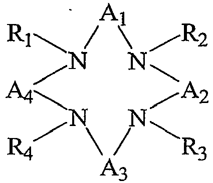 wherein A₁, A₂, A₃, and A₄, are alkyl;
and R₁, R₂, and R₃ are each independently chosen from the group consisting of: hydrogen, 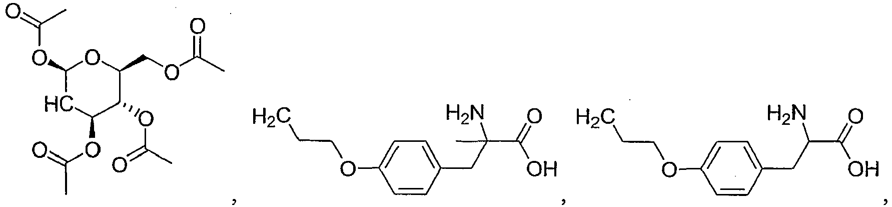 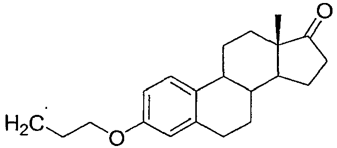 and 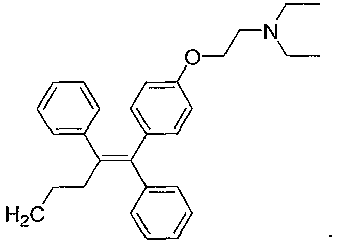 wherein R₄ is chosen from the group consisting of: 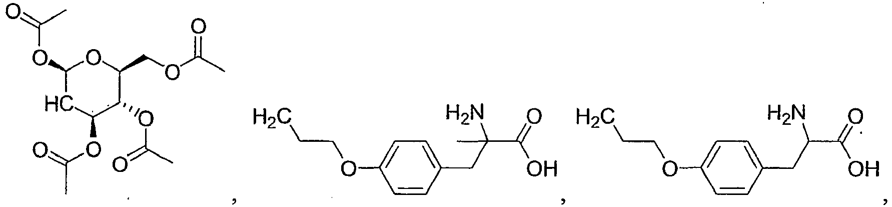 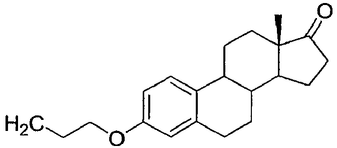 and

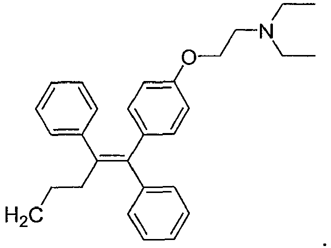

The N4 derivative may have a metal atom chelated to it *(i.e.,* the N4 derivative may be labeled with a radioisotope). The metal atom may be radioactive or non-radioactive.

### D. Radioisotope labeling

To facilitate certain embodiments involving, for example, imaging or the use of a N4 derivative as a chemotherapeutic, a radioisotope may be chelated to the N4 derivative. For example, a N4 derivative may be conjugated (preferably chelated) to ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu or ⁶²Cu.

Generally, it is believed that virtually any α, β-emitter, γ-emitter, or β, γ-emitter can be used in conjunction with the invention. Preferred α emitters include bismuth-213, astatine-211, and radium-223. Preferred β, γ-emitters include ¹⁶⁶Ho, ¹⁸⁸Re, ¹⁸⁶Re, ¹⁵³Sm, and ⁸⁹Sr. Preferred β-emitters include ⁹⁰Y and ²²⁵Ac. Preferred γ-emitters include ⁶⁷Ga, ⁶⁸Ga, ⁶⁴Cu, ⁶²Cu and ¹¹¹In. Preferred α-emitters include ²¹¹At and ²¹²Bi. It is also envisioned that paramagnetic substances, such as Gd, Mn, Cu or Fe can be chelated with N4 derivatives for use in conjunction with the present invention.

In radioimaging, the radiolabel is typically a gamma-radiation emitting radionuclide and the radiotracer is typically located using a gamma-radiation detecting camera (this process is often referred to as gamma scintigraphy). The imaged site is detectable because the radiotracer is chosen either to localize at a pathological site (termed positive contrast) or, alternatively, the radiotracer is chosen specifically not to localize at such pathological sites (termed negative contrast).

A variety of radionuclides are known to be useful for radioimaging and radioimmunotherapy, including ⁶⁷Ga/⁶⁸Ga, *^{99m}*Tc, ¹¹¹In, ¹²³I, ¹²⁵I, ¹⁶⁹Yb or ¹⁸⁶Re/¹⁸⁸Re. Due to better imaging characteristics and lower price, attempts have been made to replace or provide an alternative to ¹²³I, ¹³¹I, ⁶⁷Ga and ¹¹¹In labeled compounds with corresponding *^{99m}*Tc labeled compounds when possible. Due to favorable physical characteristics as well as extremely low price ($0.21/mCi), *^{99m}*Tc is preferred to label radiopharmaceuticals. Although it has been reported that DTPA-drug conjugate could be labeled with *^{99m}*Tc effectively (Mathias *et al*., 1997), DTPA moiety does not chelate with *^{99m}*Tc as stably as with ¹¹¹In (Goldsmith, 1997).

A number of factors must be considered for optimal radioimaging in humans. To maximize the efficiency of detection, a radionuclide that emits gamma energy in the 100 to 200 keV range is typically preferred. To minimize the absorbed radiation dose to the patient, the physical half-life of the radionuclide should be as short as the imaging procedure will allow. To allow for examinations to be performed on any day and at any time of the day, it is advantageous to have a source of the radionuclide always available at the clinical site. *^{99m}*Tc is often a preferred radionuclide because it emits gamma radiation at 140 keV, it has a physical half-life of 6 hours, and it is readily available on-site using a molybdenum-99/technetium-*99m* generator.

In certain embodiments, the N4 derivative may be labeled (e.g., chelated) with ⁶⁸Ga for PET imaging or ¹⁸⁸Re (a beta and gamma emitter) for internal radionuclide therapy. As stated above, ^{99m}Tc, ⁶⁸Ga and ¹⁸⁸Re may be obtained from generators which are accessible and affordable. When chelated with non-radioactive metals (*e.g*. copper, cobalt, platinum, iron, arsenic, rhenium, germanium), the cold (non-radioactive) N4 derivative may be used as a metallic chemotherapeutic agent. One aspect of the uniqueness of this technology is to use the same PET sulfonate precursors or SPECT iodinated agents to react with a N4 compound which is a chelator. The end product may then be chelated with metals which is easier, more accessible, and more affordable.

Technetium has a number of oxidation states: +1, +2, +4, +5, +6 and +7. When it is in the +1 oxidation state, it is called Tc MIBI. Tc MIBI is typically produced with a heat reaction (Seabold *et al.* 1999). For certain embodiments involving chelating Tc to a N4 compound or a N4 derivative, it is typically preferable that Tc is in the +4 oxidation state. This oxidation state is ideal for forming the chelate with a N4 compound or a N4 derivative. Thus, in forming a complex of radioactive technetium with the drug conjugates, the technetium complex, preferably a salt of *^{99m}*Tc pertechnetate, is typically reacted with the drug conjugates in the presence of a reducing agent.

The preferred reducing agent for use in the present invention is stannous ion in the form of stannous chloride (SnCl₂) to reduce the Tc to its +4 oxidation state. However, it is contemplated that other reducing agents, such as dithionate ion or ferrous ion may be useful in conjunction with the present invention. It is also contemplated that the reducing agent may be a solid phase reducing agent. The amount of reducing agent can be important as it is necessary to avoid the formation of a colloid. It is preferable, for example, to use from about 10 to about 100 µg SnCl₂ per about 100 to about 300 mCi of Tc pertechnetate. The most preferred amount is about 0.1 mg SnCl₂ per about 200 mCi of Tc pertechnetate and about 2 ml saline.

In addition to imaging tumors with N4 derivatives labeled with radionuclides, it is envisioned that these compounds may also be used for imaging of tissue related to other diseases, as well as diagnostics related to cancer and other diseases. For example, it is contemplated that the N4 derivatives labeled with radionuclides may be useful to image not only tumors, but also other tissue-specific conditions, such as infection, hypoxic tissue (stroke), myocardial infarction, apoptotic cells, Alzheimer's disease and endometriosis. An advantage of imaging using a N4 derivative that comprises a radiolabeled N4 compound that is conjugated to a tissue targeting ligand is that the specific binding properties of the tissue targeting ligand concentrates the radioactive signal over the area of interest.

### E. Kit for preparing radiolabelled N4 derivatives

Complexes and means for preparing such complexes may be provided in a kit form that typically includes a sealed vial containing a predetermined quantity of a N4 derivative and a sufficient amount of reducing agent to label the conjugate with a radionuclide. In some embodiments, the kit includes a radionuclide. In certain further embodiments, the radionuclide is *^{99m}*Tc. The kit may also contain conventional pharmaceutical adjunct materials such as, for example, pharmaceutically acceptable salts to adjust the osmotic pressure, buffers, preservatives, antioxidants, and the like.

In certain embodiments, an antioxidant and a transition chelator are included in the composition to prevent oxidation of the N4 derivative. In certain embodiments, the antioxidant is vitamin C (ascorbic acid). However, it is contemplated that any other antioxidant known to those of ordinary skill in the art, such as tocopherol, pyridoxine, thiamine, or rutin, may also be used. Examples of transition chelators for use in the present invention include, but are not limited to, glucoheptonate, gluconate, glucarate, citrate, and tartarate. The components of the kit may be in liquid, frozen or dry form. In certain embodiments, kit components may be provided in lyophilized form.

### III. USES FOR N4 DERIVATIVES

The N4 derivatives may also be used for prognostic purposes. It is envisioned that N4 derivatives may be administered to a patient having a tumor. It is envisioned that the use of a radiolabelled N4 compound as a labeling strategy can be effective with ligands designed for targeting disease receptors, hypoxia markers, apoptosis defects, disease cell cycles, disease functional pathways, and assessment of pharmaceutical agents effectiveness of these biochemical processes. Imaging may be performed to determine the effectiveness of the N4 derivative against a patient's specific problem relating to disease receptors, hypoxia markers, apoptosis defects, disease cell cycles, disease functional pathways, and assessment of pharmaceutical agent's effectiveness on these biochemical processes. Using this methodology physicians can quickly determine which N4 derivative will be most effective for the patient and design the corresponding therapy or mode of treatment. This methodology possesses significant advantages over methods involving first choosing a drug and administering a round of chemotherapy, which may involve months of the patient's time at a substantial physical and financial cost before the effectiveness of the cancer chemotherapeutic agent can be determined.

The present invention may also be used to monitor the progress of former patients who have sucessfully undergone chemotherapy or radiation treatment to determine if cancer has remained in remission or is metastasizing. People with a history of cancer in their family or who have been diagnosed with a gene(s) associated with cancer may undergo monitoring by health professionals using the methodology of the current invention. The methods and pharmaceutical agents of the current invention may also be used by a health professional to monitor if cancer has started to develop in a person with cancer risk factors, such as environmental exposure to carcinogens.

### IV. DRUG ASSESSMENT

Certain drug-based ligands of the present invention can be applied in measuring the pharmacological response of a subject to a drug. A wide range of parameters can be measured in determining the response of a subject to administration of a drug. One of ordinary skill in the art would be familiar with the types of responses that can be measured. These responses depend in part upon various factors, including the particular drug that is being evaluated, the particular disease or condition for which the subject is being treated, and characteristics of the subject. Radiolabeled agents can be applied in measuring drug assessment.

### V. PHARMACEUTICAL PREPARATIONS

Pharmaceutical compositions of the present invention comprise an effective amount of an N4 derivative of the present invention dissolved or dispersed in a pharmaceutically acceptable carrier. The phrases "pharmaceutical" or "pharmacologically acceptable" refer to molecular entities and compositions that do not produce an adverse, allergic or other untoward reaction when administered to an animal, such as, for example, a human, as appropriate. The preparation of a pharmaceutical composition that contains at least one N4 derivative, such as a radiolabeled N4 derivative, or additional active ingredient will be known to those of skill in the art in light of the present disclosure, as exemplified by Remington's Pharmaceutical Sciences, 18th Ed. Mack Printing Company, 1990 . Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington's Pharmaceutical Sciences, 18th Ed. Mack Printing Company, 1990, pp. 1289-1329). Except insofar as any conventional carrier is incompatible with the active ingredient, its use in the therapeutic or pharmaceutical compositions is contemplated.

The N4 derivatives of the present invention may comprise different types of carriers depending on whether it is to be administered in solid, liquid or aerosol form, and whether it needs to be sterile for such routes of administration such as injection. The present invention can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctival, intravesicularlly, mucosally, intrapericardially, intraumbilically, intraocularally, orally, topically, locally, injection, infusion, continuous infusion, localized perfusion bathing target cells directly, via a catheter, via a lavage, in lipid compositions (e.g., liposomes), or by other method or any combination of the forgoing as would be known to one of ordinary skill in the art (see, for example, Remington's Pharmaceutical Sciences, 18th Ed. Mack Printing Company, 1990 ).

The actual dosage amount of a composition of the present invention administered to a patient can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

In certain embodiments, pharmaceutical compositions may comprise, for example, at least about 0.1% of a N4 derivative. In other embodiments, the active compound may comprise between about 2% to about 75% of the weight of the unit, or between about 25% to about 60%, for example, and any range derivable therein. In other non-limiting examples, a dose may also comprise from about 0.1 mg/kg/body weight, 0.5 mg/kg/ body weight, 1 mg/kg/body weight, about 5 mg/kg/body weight, about 10 mg/kg/body weight, about 20 mg/kg/body weight, about 30 mg/kg/body weight, about 40 mg/kg/body weight, about 50 mg/kg/body weight, about 75 mg/kg/body weight, about 100 mg/kg/body weight, about 200 mg/kg/body weight, about 350 mg/kg/body weight, about 500 mg/kg/body weight, about 750 mg/kg/body weight, to about 1000 mg/kg/body weight or more per administration, and any range derivable therein. In non-limiting examples of a derivable range from the numbers listed herein, a range of about 10 mg/kg/body weight to about 100 mg/kg/body weight, etc., can be administered, based on the numbers described above.

In any case, the composition may comprise various antioxidants to retard oxidation of one or more component. Additionally, the prevention of the action of microorganisms can be brought about by preservatives such as various antibacterial and antifungal agents, including, but not limited to parabens (e.g., methylparabens, propylparabens), chlorobutanol, phenol, sorbic acid, thimerosal or combinations thereof.

The N4 derivative may be formulated into a composition in a free base, neutral or salt form. Pharmaceutically acceptable salts include the salts formed with the free carboxyl groups derived from inorganic bases such as for example, sodium, potassium, ammonium, calcium or ferric hydroxides; or such organic bases as isopropylamine, trimethylamine, histidine or procaine.

In embodiments where the composition is in a liquid form, a carrier can be a solvent or dispersion medium comprising, but not limited to, water, ethanol, polyol (e.g., glycerol, propylene glycol, liquid polyethylene glycol, *etc.),* lipids *(e.g.,* triglycerides, vegetable oils, liposomes) and combinations thereof. The proper fluidity can be maintained, for example, by the use of a coating, such as lecithin; by the maintenance of the required particle size by dispersion in carriers such as, for example, liquid polyol or lipids; by the use of surfactants such as, for example, hydroxypropylcellulose; or combinations thereof such methods. In many cases, it will be preferable to include isotonic agents, such as, for example, sugars, sodium chloride or combinations thereof.

Sterile injectable solutions are prepared by incorporating the N4 derivative in the required amount of the appropriate solvent with various amounts of the other ingredients enumerated above, as required, followed by filtered sterilization. Generally, dispersions are prepared by incorporating the various sterilized active ingredients into a sterile vehicle which contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

In particular embodiments, prolonged absorption of an injectable composition can be brought about by the use in the compositions of agents delaying absorption, such as, for example, aluminum monostearate, gelatin or combinations thereof.

### VI. COMBINATIONAL THERAPY

It is an aspect of this invention that N4 derivatives, such as a radiolabeled N4 derivative, of the present invention can be used in combination with another agent or therapy method, preferably another cancer treatment. The N4 derivative may precede or follow the other agent treatment by intervals ranging from minutes to weeks. In embodiments where the other agent and expression construct are applied separately to the cell, one would generally ensure that a significant period of time did not expire between the time of each delivery, such that the agent and expression construct would still be able to exert an advantageously combined effect on the cell. For example, in such instances, it is contemplated that one may contact the cell, tissue or organism with two, three, four or more modalities substantially simultaneously *(i.e.,* within less than about a minute) with the N4 derivative. In other aspects, one or more agents may be administered within about 1 minute, about 5 minutes, about 10 minutes, about 20 minutes about 30 minutes, about 45 minutes, about 60 minutes, about 2 hours, about 3 hours, about 4 hours, about 5 hours, about 6 hours, about 7 hours about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, about 24 hours, about 25 hours, about 26 hours, about 27 hours, about 28 hours, about 29 hours, about 30 hours, about 31 hours, about 32 hours, about 33 hours, about 34 hours, about 35 hours, about 36 hours, about 37 hours, about 38 hours, about 39 hours, about 40 hours, about 41 hours, about 42 hours, about 43 hours, about 44 hours, about 45 hours, about 46 hours, about 47 hours, to about 48 hours or more prior to and/or after administering the N4 derivative. In certain other embodiments, an agent may be administered within of from about 1 day, about 2 days, about 3 days, about 4 days, about 5 days, about 6 days, about 7 days, about 8 days, about 9 days, about 10 days, about 11 days, about 12 days, about 13 days, about 14 days, about 15 days, about 16 days, about 17 days, about 18 days, about 19 days, about 20, to about 21 days prior to and/or after administering the N4 derivative. In some situations, it may be desirable to extend the time period for treatment significantly, however, where several weeks (e.g., about 1, about 2, about 3, about 4, about 5, about 6, about 7 or about 8 weeks or more) lapse between the respective administrations.

Various combinations may be employed, the N4 derivative is "A" and the secondary agent, which can be any other therapeutic agent, is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of the therapeutic expression constructs of the present invention to a patient will follow general protocols for the administration of chemotherapeutics, taking into account the toxicity, if any, of the vector. It is expected that the treatment cycles would be repeated as necessary. It also is contemplated that various standard therapies, as well as surgical intervention, may be applied in combination with the N4 derivative. These therapies include but are not limited to chemotherapy, radiotherapy, immunotherapy, gene therapy and surgery.

### A. Chemotherapy

Cancer therapies also include a variety of combination therapies with both chemical and radiation based treatments. Combination chemotherapy include, for example, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein tansferase inhibitors, cisplatinum, 5-fluorouracil, vincristin, vinblastin and methotrexate, or any analog or derivative variant of the foregoing.

### B. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely and depend on the half-life of the isotope, the strength and type of radiation emitted, and the uptake by the neoplastic cells. The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

### C. Immunotherapy

Immunotherapeutics, generally, rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionucleotide, ricin A chain, cholera toxin, pertussis toxin, *etc.)* and serve merely as a targeting agent. Alternatively, the effector may be a lymphocyte carrying a surface molecule that interacts, either directly or indirectly, with a tumor cell target. Various effector cells include cytotoxic T cells and NK cells.

Immunotherapy could thus be used as part of a combined therapy, possibly in conjunction with gene therapy. The general approach for combined therapy is discussed below. Generally, the tumor cell must bear some marker that is amenable to targeting, *i.e*., is not present on the majority of other cells. Many tumor markers exist and any of these may be suitable for targeting in the context of the present invention. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, *erb* B and p155.

### D. Gene therapy

In yet another embodiment, the secondary treatment is a secondary gene therapy in which a therapeutic polynucleotide is administered before, after, or at the same time a first therapeutic agent. Delivery of the therapeutic agent in conjunction with a vector encoding a gene product will have a combined anti-hyperproliferative effect on target tissues.

### E. Surgery

Approximately 60% of persons with cancer will undergo surgery of some type, which includes preventative, diagnostic or staging, curative and palliative surgery. Curative surgery is a cancer treatment that may be used in conjunction with other therapies, such as the treatment: disclosed herein chemotherapy, radiotherapy, hormonal therapy, gene therapy, immunotherapy and/or alternative therapies. Curative surgery includes resection in which all or part of cancerous tissue is physically or partially removed, excised, and/or destroyed. Tumor resection refers to physical removal of at least part of a tumor. In addition to tumor resection, treatment by surgery includes laser surgery, cryosurgery, electrosurgery, and miscopically controlled surgery (Mohs' surgery). It is further contemplated that the present invention may be used in conjunction with removal of superficial cancers, precancers, or incidental amounts of normal tissue.

### VII. EXAMPLES

### EXAMPLE 1 (Reference)

### Protection of N4-compounds for N-mono substitutes

### A. Protection of cyclam with ethyl trifluoroacetate

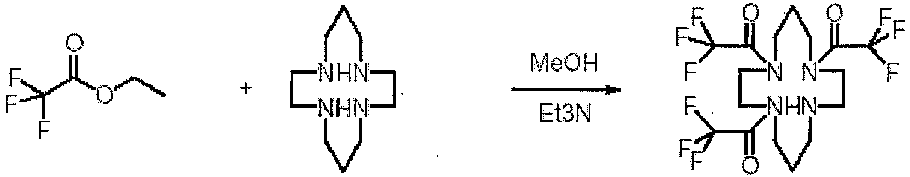

4.006 g (20 mmol) of cyclam(1,4,8,11-Tetraazacyclotetradecane) put into the solution of 2.79 mL of triethyl aminein 15 mL of dried Methanol. 6.92 mL of ethyl trifluoroacetate was added dropwise to the upper solution at room temperature with stirring. The addition continued over a period of 5 min. The homogeneous reaction mixture was cooled with an ice-water bath to control the mild exothermic. Stirring was continued under N2 for 5 hours. Volatiles were removed in vacuo. The residue was passed through a small silica-gel plug(25g) and eluted with 100% EtOAc. The eluted solvent was concentrated to give the product as a white foam (8.972g, 95% yield).

### B. Protection of cyclen with ethyl trifluoroacetate

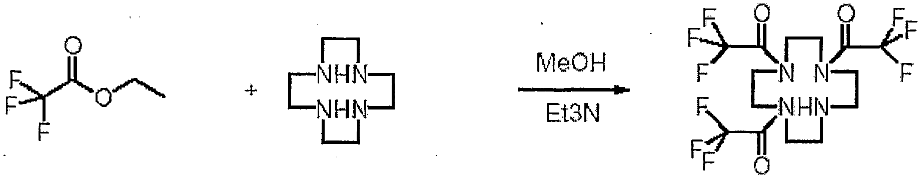

3.445 mg (20 mmol) of cyclen (1,4,7,10-Tetraazacyclododecane) instead of cyclam was used in this reaction following the above (Example 1, A) method. (8.276g, 93% yield).

### C. Protection of cyclal with ethyl trifluoroacetate

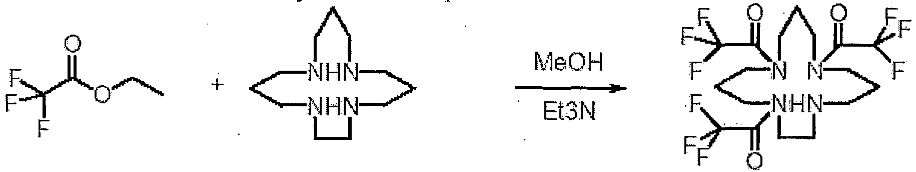

4.287g (20 mmol) of cyclal (1,4,8,12-Tetraazacyclopentadecane) instead of cyclam was used in this reaction following the above (Example 1, A) method. (9.227g, 95% yield).

### EXAMPLE 2 (Reference)

### Preparation of sulfonated and iodinated tyrosine-derivatives

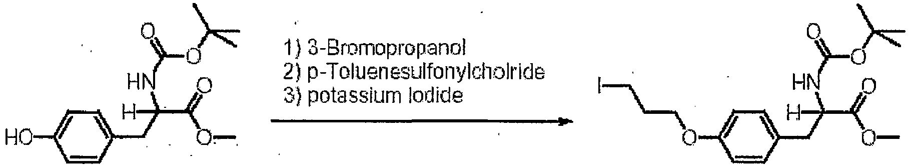

### A. O-alkylation of tyrosine with 3-bromopropanol

2953.3 mg (10 mmol) of N-(tert-buthoxycarbonyl)-L-tyrosine methyl eater (below Boc-Tyr) in 30mL of methanol(anhydrous) solution was added into 50 mL of methanol solution containing of 540.2 mg (10 mmol) of sodiummethoxide. 1363 µL (15 mmol) of 3-bromopropanol was added to the upper Boc-Tyr solution. The mixture was stirred at 70°C for 6 hours after at room temperature for 20 min under nitrogen atmosphere. The mixture was dissolved in 20 mL of ethyl acetate after evaporated under reduced pressure in order to remove volatiles. The organic layer was washed with water (2 X 20 mL), dried with magnesium sulfate anhydrous and removed the solvent using rotary evaporator. Clear liquid, turning to white solid, hydroxypropyl-Boc-Tyr (below HOPr-Boc-Tyr), 2.9158g(82.5% yield) was yielded through column chromatography using gradient hexane-ethyl acetate system.(10:1 to 1:1).

### B. Tosylation of 3-hydroxypropyl-Boc-Tyr

1413.6 mg (4.0 mmol) of hydroxypropyl-Boc-Tyr (below HOPr-Boc-Tyr) in 10 ml of pyridine(anhydrous) was poured into the solution of 1143.9 mg (6.0 mmol) of p-Toluenesulfonyl chloride in 20 mL of pyridine (anhydrous) with stirring in ice-water bath under nitrogen atmosphere. The mixture was placed in a refrigerator overnight. The reaction mixture can be followed by the development of color, followed by filter separation of pyridine hydrochloride. The filtrate was evaporated under reduced pressure in order to remove pyridine. White solid, 1.8123g(87.9% yield) was yielded through column chromatography using gradient hexane-ethyl acetate system.(10:1 to 2:1)

### C. Synthesis of 3-Iodopropyl-Boc-Tyr (below I-Pr-Boc-Tyr)

Potassium iodide 1992.1 mg (12 mmol) was poured into the solution of TsO-Pr-Boc-Tyr 1522.8 mg (3.0 mmol) in 15 ml of acetonitrile (anhydrous). The mixture was not fully dissolved in solvent and was allowed to reflux for 2 hours. The solid was filtered off and filtered solution was evaporated to remove acetonitrile. The residue was isolated by column chromatography using gradient hexane : ethyl acetate system (10:1 to 10:4). Clear liquid 1324.5 mg was gained (95.3% yield).

### EXAMPLE 3

### Preparation of sulfonated and iodinated alpha-methyltyrosine-derivatives

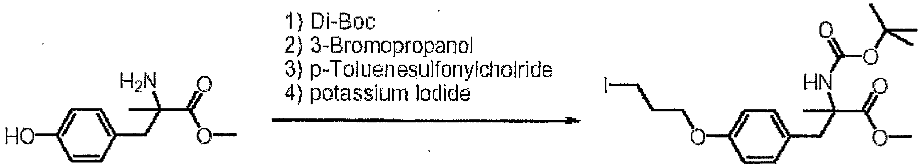

### A. N-Protection of alpha-methyl tyrosine

Di-tert-butyl dicarbonate 13.095 g (60 mmol) was added to the solution of alpha-methyltyrosine (below AMT) 8.370 g (40 mmol) and triethyl amine(Anhydrous) 11.2 mL (80 mmol) in 40 mL of DMF (Anhydrous). The mixture was evaporated under reduced pressure followed by filtering to get rid of solid after stirring overnight at room temperature. White solid (below Boc-AMT) 11.217 g (90.6% yield) was gained through column isolation of the residue using gradient hexane-ethyl acetate system (10:1 to 10:7).

### B. O-alkylation of Boc-AMT with 3-bromopropanol

3.094 g (10 mmol) of Boc-AMT was used and followed using the above (Example 2, B) method. Clear liquid, turning to white solid (below HO-Pr-Boc-AMT), 3.289 g (89.5% yield) was yielded through column chromatography using gradient hexane-ethyl acetate system.(10:1 to 10:5)

### C. Tosylation of 3-HO-Pr-Boc-AMT

2.940 g (8.0 mmol) of HO-Pr-Boc-AMT was used and followed as upper 2-3) method. White solid (below TsO-Pr- Boc-AMT), 3.493g(83.7% yield) was yielded through column chromatography using gradient hexane-ethyl acetate system (10:1 to 10:5).

### D. Synthesis of 3-Iodopropyl-Boc-Tyr (below I-Pr-Boc-Tyr)

3.130 g (6.0 mmol) of TsO-Pr-Boc-AMT was used and followed as upper 2-4) method. Clear liquid (below I-Pr- Boc-AMT), 2.801g(97.8% yield) was yielded through column chromatography using gradient hexane-ethyl acetate system.(10:1 to 10:4).

### EXAMPLE 4 (Reference)

### Preparation of sulfonated and iodinated Tamoxifen-derivatives

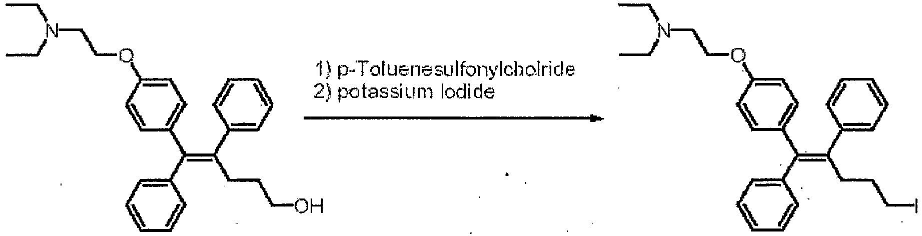

### A. Tosylation of 4-hydroxymethyl-N,N-diethyl Tamoxifen

1.289g(8.0mmol) of 4-hydroxymethyl-N,N-diethyl Tamoxifen(below HO-TMX) was used and followed as upper 2-3) method. Pale yellow liquid(below TsO-TMX), 1.445g(82.5% yield) was yielded through column chromatography using gradient hexane : ethyl ether : triethyl amine = 100: 100: 5 to 100: 100: 20)

### B. Synthesis of 4-Iodomethyl-N,N-diethyl

1.168g(2.0mmol) of I-TMX was used and followed as the above (Example 2, D) method. Clear liquid (below I-TMX), 1.058g(98.1% yield) was yielded after passing short column chromatography using gradient hexane : ethyl ether : triethyl amine = 100: 100: 1 to 100: 100: 10)

### EXAMPLE 5 (Reference)

### Preparation of sulfonated and iodinated Estrone-derivatives

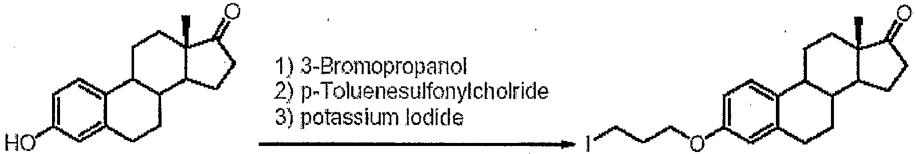

### A. O-alkylation of Estrone with 3-bromopropanol

2.703g(10mmol) of Estrone was used and followed as upper 2-2) method. Clear liquid, turning to white solid(below HO-Pr-EST), 2.405g(73.2% yield) was yielded through column chromatography using gradient hexane-ethyl acetate system.(10:1 to 10:5)

### B. Tosylation of HO-Pr-EST

1.971g(6.0mmol) of HO-Pr-EST was used and followed as upper 2-3) method. White solid (below TsO-Pr-EST), 2.253g(77.8% yield) was yielded through column chromatography using gradient hexane-ethyl acetate system(10:1 to 10:5)

### C. Synthesis of 3-Iodopropyl-EST

1.930g(4.0mmol) of TsO-Pr-EST was used and followed as upper 2-4) method. Clear liquid (below I-Pr-EST) 1.720g(98.1% yield) was yielded through column chromatography using gradient hexane-ethyl acetate system.(10:1 to 10:4)

### EXAMPLE 6 (Reference)

### Reaction of cyclal with 1,3,4,6-Tetra-O-acetyl-2-O-trifluoromethanesulfonyl-beta-Dmannopyranose (precursor for FDG synthesis)

### A. Example of N,N',N",N'''-tetra-substituted cyclal-DG

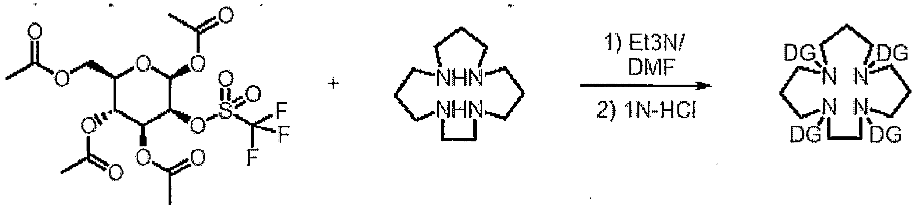

1,3,4,6-Tetra-O-acetyl-2-O-trifluoromethanesulfonyl-beta-D-mannopyranose 200 mg (0.416 mmol) put into the solution of cyclal 22.3mg (0.104 mmol) and triethyl amine 84.2 mg, 116 micromL(0.832mmol) in DMF(Anhydrous) 10mL. The mixture was stirred at 50°C for 16 hours under nitrogen atmosphere and evaporated to remove volatiles. The residue put into 1,4-dioxane 6mL, then white precipitate came out. The solid was thrown away through filtering. 1ml 4N-HCl in 1,4-dioxane solution was added drop by drop to filtrate solution, then pale brown powder precipitated. The solid was collected with filtering and dried under lyophilizer.

The solid was dissolved in 1N-hydrochloric acid aq. soln 3mL and stirred for 30min. 1N-NaHCO3 was added to upper solution to pH = ca 9. The solution was purified with membrane (MW cut off < 500), and evaporated under Lyophilizer. Pale brown solid 63.2mg(67.2% yield) was collected.

### B. Example of N,N',N"-tri-substituted cyclal-DG

1,3,4,6-Tetra-O-acetyl-2-O-trifluoromethanesulfonyl-beta-D-mannopyranose 200mg(0.416mmol) put into the solution of cyclal 29.6mg (0.138mmol) and triethyl amine 84.2mg, 116micromL(0.832mmol) in IDMF(Anhydrous) 10mL. The mixture was stirred at 50deg for 16hours under nitrogen atmosphere and evaporated to remove volatiles. The residue put into 1,4-dioxane 6mL, then white precipitate came out. The solid was thrown away through filtering. 1ml 4N-HCl in 1,4- dioxane solution was added drop by drop to filtrate solution, then pale brown powder precipitated.

The solid was collected with filtering and dried under lyophilizer. The solid was dissolved in 1N-hydrochloric acid aq. soln 3mL and stirred for 30min. 1N-NaHCO3 was added to upper solution to pH = ca 9. The solution was purified with membrane (MW cut off < 500), and evaporated under Lyophilizer. Pale brown solid 58.8mg(57.4% yield) was collected.

### C. Example of N,N'-di-substituted cyclal-DG

1,3,4,6-Tetra-O-acetyl-2-O-trifluoromethanesulfonyl-beta-D-mannopyranose 200mg(0.416mmol) put into the solution of cyclal 44.6mg (0.208mmol) and triethyl amine 84.2mg, 116micromL(0.832mmol) in DMF(Anhydrous) 10mL. The mixture was stirred at 50deg for 16hours under nitrogen atmosphere and evaporated to remove volatiles. The residue put into 1,4-dioxane 6mL, then white precipitate came out. The solid was thrown away through filtering. 1ml 4N-HCl in 1,4-dioxane solution was added drop by drop to filtrate solution, then pale brown powder precipitated. The solid was collected with filtering and dried under lyophilizer.

The solid was dissolved in 1N-hydrochloric acid aq. soln 3mL and stirred for 30min. 1N-NaHCO3 was added to upper solution to pH = ca 9. The solution was purified with membrane (MW cut off < 500), evaporated under Lyophilizer. The residue dissolved in minimum water. Pale brown solid 23.9mg(19.8% yield) was collected through Lyophilizer after Sephadex G-75 isolation.

### D. Example of N-mono substituted cyclal-DG

1,3,4,6-Tetra-O-acetyl-2-O-trifluoromethanesulfonyl-beta-D-mannopyranose 200 mg (0.416 mmol) put into the solution of N,N',N"-tris (trifluoroacetyl)-cyclal(from Example 1, C) 209mg (0.416mmol) and triethyl amine 84.2mg, 116micromL (0.832mmol) in DMF (Anhydrous) 10mL. The mixture was stirred at 50°C for 6 hours under nitrogen atmosphere and evaporated to remove volatiles.

The residue was put into 1,4-dioxane 6mL, then white precipitate came out. The solid was thrown away through filtering. 1 ml 4N-HCl in 1,4-dioxane solution was added drop by drop to filtrate solution, then pale brown powder precipitated. The solid collected with filtering was dissolved in 1N-hydrochloric acid aq. soln 3 mL and stirred for 30 min. 1N-NaHCO3 was added to upper solution to pH = ca 9. The solution was evaporated under Lyophilizer and dissolved in minimum water. White solid 123.3 mg(78.7% yield) was collected through Lyophilizer after Sephadex G-25 isolation.

### EXAMPLE 7

### Reaction of cyclal with iodinated alpha methyltyrosine (AMT)

Similar reaction conditions could be used to prepare other cyclal-targeting agents conjugates.

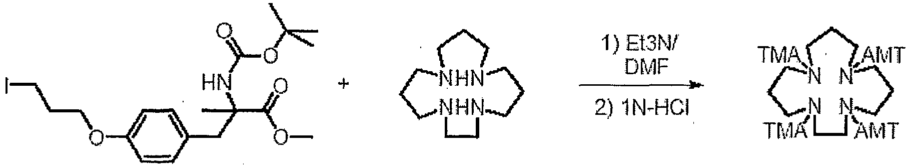

### A. Example of N,N',N",N"'-tetra-substituted AMT

I-AMT 286.4mg (0.6mmol) put into the solution of cyclal 32.2 mg (0.15mmol) and triethyl amine 83.6µL (0.6mmol) in DMF (Anhydrous) 10mL. The mixture was stirred at 70°C for 16 hours under nitrogen atmosphere and evaporated to remove volatiles. 1N-hydrochloric acid aq. soln 5 mL poured into the solution of the evaporating residue in ethanol 5 mL. The reaction mixture was heated at 60°C for 30 min without condenser and cooled. 1N-NaHCO3 was added to upper solution to pH = ca 9. The solvent was removed under reduced pressure and the residue dissolved in minimum water. White solid 91.3mg(52.7% yield) was collected through Lyophilizer after Sephadex G-75 isolation.

### B. Example of N,N',N"-tri-substituted AMT

I-AMT 286.4mg(0.6mmol) put into the solution of cyclal 42.9mg(0.2mmol) and triethyl amine 83.6micrlL(0.6mmol) in DMF(Anhydrous) 10mL. The mixture was stirred at 70deg for 16hours under nitrogen atmosphere and evaporated to remove volatiles.

1N-hydrochloric acid aq.soln 5mL poured into the solution of the evaporating residue in ethanol 5mL. The reaction mixture was heated at 60deg for 30min without condenser and cooled. 1N-NaHCO3 was added to upper solution to pH = ca 9. The solvent was removed under reduced pressure and the residue dissolved in minimum water. White solid 76.7mg (41.7% yield) was collected through Lyophilizer after Sephadex G-75 isolation.

### C. Example of N,N'-di-substituted AMT

I-AMT 286.4mg(0.6mmol) put into the solution of cyclal 64.3 mg (0.3mmol) and triethyl amine 83.6 µL (0.6mmol) in DMF(Anhydrous) 10mL. The mixture was stirred at 70deg for 16hours under nitrogen atmosphere and evaporated to remove volatiles. 1N-hydrochloric acid aq.soln 5mL poured into the solution of the evaporating residue in ethanol 5mL. The reaction mixture was heated at 60deg for 30min without condenser and cooled. 1N-NaHCO3 was added to upper solution to pH = ca 9. The solvent was removed under reduced pressure and the residue dissolved in minimum water. White solid 52.3mg(25.9% yield) was collected through Lyophilizer after Sephadex G-75 isolation.

### D. Example of N-mono substituted AMT

I-AMT 286.4mg(0.6mmol) put into the solution of N,N',N"-tris(trifluoroacetyl)-cyclal(from example 1-3) 301.4mg(0.6mmol) and triethyl amine 83.6micrlL(0.6mmol) in DMF(Anhydrous) 10mL. The mixture was stirred at 70deg for 6hours under nitrogen atmosphere and evaporated to remove volatiles. 1N-potassium carbonate 2mL poured into the solution of evaporating residue in methanol 5ml and allowed to keep at 40deg for 1hour.

1N-hydrochloric acid aq.soln 9mL add to upper solution. The reaction mixture was heated at 60deg for 30min without condenser and cooled. 1N-NaHCO3 was added to upper solution to pH = ca 9. The solvent was removed under reduced pressure and the residue dissolved in minimum water. White solid 233.4mg (86.5% yield) was collected through Lyophilizer after Sephadex G-25 isolation.

### EXAMPLE 8

### Reaction of cyclal with sulfonated alpha methyltyrosine (TsO-AMT)

Similar reaction conditions to the ones presented herein may be used to prepare other cyclal-targeting agents conjugates.

### A. Example of N,N',N",N"'-tetra-substituted AMT

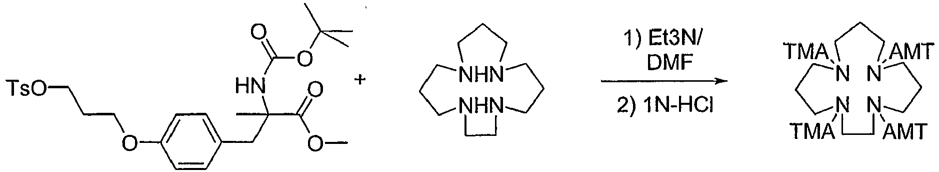

TsO-AMT 313 mg (0.6mmol) put into the solution of cyclal 32.2 mg (0.15mmol) and triethyl amine 167.2µL (1.2mmol) in DMF(Anhydrous) 10mL. The reaction was followed upper example of 7-1). White solid 72.3mg(41.7% yield) was collected.

### B. Example of N,N',N''-tri-substituted AMT

TsO-AMT 313mg(0.6mmol) put into the solution of cyclal 42.9mg(0.2mmol) and triethyl amine 167.2micr1L(1.2mmol) in DMF(Anhydrous) 10mL. The reaction was followed upper example of 7-2). White solid 56.7mg (30.8% yield) was collected.

### C. Example of N,N'-di-substituted AMT

TsO-AMT 313.0mg (0.6mmol) put into the solution of cyclal 64.3 mg (0.3mmol) and triethyl amine 167.2 µL(1.2mmol) in DMF(Anhydrous) 10mL. The reaction was followed upper example of 7-3). White solid 45.4mg(22.1% yield) was collected.

### D. Example of N-mono substituted AMT

TsO-AMT 313.0mg(0.6 mmol) put into the solution of N,N',N"-tris(trifluoroacetyl)-cyclal(from example 1-3) 301.4 mg (0.6mmol) and triethyl amine 167.2 µL (1.2mmol) in DMF (Anhydrous) 10mL. The reaction was followed upper example of 7-4). White solid 197.2 mg (73.1 % yield) was collected.

### EXAMPLE 9

### Imaging using N4 derivatives

### A. Materials and Methods

### Reaction of cyclam with tetraacetate mannose conjugates (N4-DG-cyclam)

1,3,4,6-Tetra-O-acetyl-2-O-trifluoromethanesulfonyl-beta-D-mannopyranose (300 mg, 0.625 mmol) in 5 mL of DMF was added to the mixture of 1,4,8,12-tetraazacyclopentacecane (N4) (250.2 mg, 1.237 mmol) and triethylamine (174 microL, 1.249 mmol) in 5 mL of DMF. The reaction mixture was stirred at room temperature for 6 hrs. The reaction solvent was evaporated to dryness at 40-45°C under high vacuum. 1,4-Dioxane solution (10 mL) was then added. The precipitate was filtered. Hydrochloric acid (4N) in 1,4-dioxane (2 mL, 8 mmol) solution was added. The mixture was cooled in an ice-bath. The mixture was filtered through a Buchner funnel and washed with diethyl ether (2x5 mL). The filtrate was evaporated to dryness, yielded white solid 383.1 mg (90.8%). 1H- NMR of N4-DG δ(ppm)8.50 (s, 1H), 3.98-4.01 (m, 1H), 3.76 (s, 2H), 3.54-3.60 (m, 9H), 3.38-3.45 (m, 8H), 3.31-3.37 (m, 1H), 3.18-3.22 (m, 1H), 2.02-2.31 (m, 4H), 2.15 (s, 12H). 13C- NMR of N4-DG δ(ppm) 197.3, 175.2, 170.4, 165.6, 67.0, 66.8, 66.4, 51.7, 45.3, 44.0, 43.6, 43.2, 42.9, 42.5, 41.9, 38.6, 37.5, 37.3, 31.8, 19.5, 19.3, 14.5. The synthetic scheme is shown in FIG. 1.

### Radiolabeling of N4-DG (N4-DG-cyclam)

N4-DG (5mg) was dissolved in 0.2 ml water. Tin(II) chloride solution (0.1 ml, 1mg/ml) was added. Sodium pertechnetate (Na^{99m}TcO₄, 37-370 MBq, Mallinckrodt, Houston, TX) was added. Finally, water was added to this solution to adjust the volume to 1 ml. Radiochemical purity was determined by TLC (ITLC SG, Gelman Sciences, Ann Arbor, MI) eluted with methanol : ammonium acetate (1:4). From radio-TLC analysis (Bioscan, Washington, DC), the radiochemical purity was more than 97%.

For ⁶⁸Ga-labeling, ⁶⁸Ga was eluted from a ⁶⁸Ge/⁶⁸Ga generator (Isotope Products Laboratories, Valencia, CA) using 1N HCL. The acidic solution was evaporated to dryness with either GaCl₃ carrier added or no carrier added. The soultion was reconstituted in water. N4-DG (5mg) dissolved in 0.2 ml water was then added to the radioactive solution.

### In Vitro Cellular Uptake of ^{99m}Tc-N4-DG (N4-DG-cyclam) and N4-DG-cyclal)

Two different cancer cell lines (human lung NSCLC A549, breast 13762) were used for cellular uptake assays. The cell lines were obtained from American Type Culture Collection (Rockville, MD). The cells were plated to 12 wells tissue culture plate that contained 50,000 per each well. 4 µCi (0.148 MBq) of ^{99m}Tc- and ⁶⁸Ga-N4-DG or N4 (0.1 mg/well) was added to each well. Cells were incubated with radiotracers at 37°C at different time intervals. After incubation, cells were washed with ice-cold phosphate-buffered saline (PBS) twice and trypsinized with 0.5 ml of trypsin solution. Then cells were collected and the radioactivity was measured by gamma counter. Data are expressed in mean±SD percent uptake ratio of three measurements.

### Biodistribution of ^{99m}Tc-N4-DG in Breast Tumor-bearing Rats

The animals were housed in The University of Texas M. D. Anderson Cancer Center facility. All protocols involving animals (rats and rabbits [see below]) are approved by the M. D. Anderson Animal Use and Care Committee. Fischer-344 Rats (150±25g) (Harlan Sprague-Dawley, Indianapolis, IN) (n=18) were inoculated subcutaneously with rat breast adenocarcinoma cells (10⁶ cells/rodent) into the lumber region in legs using 25-gauge needles. The studies were performed 12 to 15 days after inoculation. Tumor sizes approximately 1 cm were measured. Biodistribution studies using ^{99m}Tc-N4-DG was conducted. The rodents were divided into three groups, each group representing a time interval (0.5, 2, and 4 hrs, n=3/time point) and containing total 9 rodents per compound. The injection activity was 25±0.5 µCi (0.925±0.019 MBq)/rat. The injected mass of ^{99m}Tc-N4-DG was 0.1 mg/rodent. Following administration of the radiotracers, the rats were sacrificed and the selected tissues were excised, weighed and counted for radioactivity. The biodistribution of tracer in each sample was calculated as percentage of the injected dose per gram of tissue wet weight (%ID/g). Tumor/nontarget tissue count density ratios were calculated from the corresponding %ID/g values.

### Scintigraphic Imaging Studies

Female Fischer 344 rats (150±25 g) (Harlan Sprague-Dawley, Indianapolis, IN) were inoculated subcutaneously with 0.1 ml of mammary tumor cells from the 13762 tumor cell line suspension (10⁶ cells/rat, a tumor cell line specific to Fischer rats) into the hind legs. Imaging studies were performed 12 to 15 days after inoculation. Tumor sizes approximately 1-1.5 cm were measured. Scintigraphic images were obtained using a M-camera from Siemens Medical Systems (Hoffman Estates, IL). The camera was equipped with a low-energy parallel-hole collimator. The field of view is 53.3 cm x 38.7 cm. The intrinsic spatial resolution is 3.2 mm and the pixel size is 19.18 mm (32x32, zoom = 1) to 0.187 mm (1024x1024, zoom = 3.2). With a low-energy, high-resolution collimator (as required with ^{99m}Tc), the system is designed for a planar sensitivity of at least 172 counts/minute (cpm)/µCi and spatial resolution of 4-20 mm. uPET was used for PET imaging studies (0.5 mCi/rat).

Planar scintigraphy was obtained at immediate, 0.5-4 hrs after i.v. injection of ^{99m}Tc-N4-DG or ^{99m}Tc-N4 (0.3 mCi/rat; 0.1mg mass/rabbit). To compare the radiotracer accumulation, ROIs (region of interest in counts per pixel) were determined. The ROIs count between tumor and muscle was used to calculate tumor-to-nontumor ratios.

### B. Results

### In Vitro Cellular Uptake Studies

There was an increased uptake of ^{99m}Tc- or ^{68Ga}-N4-DG or ^{99m}Tc-N4-AMT as a function of incubation time in the cancer cell lines tested (FIG. 2, FIG. 3, FIGS. 4A-C). Uptake of ^{99m}Tc-N4 as the control group was less that 0.5% at any time point.

### Biodistribution and Scintigraphic Imaging Studies

Biodistribution of ^{99m}Tc-N4-DG in tumor-bearing rats showed increased tumor-to-tissue count density ratios as a function of time (Table2). Planar images of tumor-bearing animal models confirmed that the tumors could be visualized clearly with ^{99m}Tc- or ^{68Ga}-N4-DG (FIGS. 5A-B, FIG. 6, FIG, 7) and N4-AMT (FIG. 9, FIG. 10). Computer outlined region of interest (ROI) showed that tumor/background ratios in ^{99m}Tc-N4-DG group were increased as a function of time (FIG. 8). The optimal imaging time was 1hr in a rat model.

**TABLE 2. Biodistribution of ^{99m}Tc-N4-DG₂-(Cyclam) in Breast Tumor-Bearing Rats**

| % of injected dose per gram of tissue weight (n=3/time, interval, iv) | | | |
|---|---|---|---|
| | 30 MIN | 2 HOURS | 4 Hours |
| BLOOD | 4.102 ±0.560 | 1.185 ±0.154 | 0.984 ±0.034 |
| HEART | 0.847 ±0.069 | 0.306 ±0.017 | 0.253 ±0.018 |
| LUNG | 3.659 ±0.212 | 2.368 ±0.050 | 3.196 ±0.395 |
| LIVER | 20.959 ±3.548 | 24.282 ±0.723 | 26.653 ±2.338 |
| SPLEEN | 8.535 ±0.886 | 16.647 ±3.310 | 11.962 ±0.655 |
| KIDNEY | 6.995 ±0.464 | 7.512 ±0.643 | 8.405 ±0.146 |
| INTESTINE | 0.626 ±0.147 | 0.454 ±0.124 | 0.256 ±0.033 |
| UTERUS | 0.575 ±0.067 | 0.294 ±0.032 | 0.230 ±0.002 |
| MUSCLE | 0.122 ±0.021 | 0.060 ±0.007 | 0.048 ±0.002 |
| TUMOR | 0.624 ±0.050 | 0.345 ±0.019 | 0.274 ±0.020 |
| THYROID | 1.285 ±0.298 | 0.485 ±0.075 | 0.314±0.031 |
| STOMACH | 0.547 ±0.033 | 0.331 ±0.038 | 0.216 ±0.003 |
| T/MUSCLE | 5.348 ±1.347 | 6.010 ±1.111 | 5.723 ±0.079 |
| T/BLOOD | 0.157 ±0.033 | 0.297 ±0.023 | 0.279 ±0.026 |
| H/BLOOD | 0.208 ±0.012 | 0.264 ±0.023 | 0.257 ±0.010 |
| H/MUSCLE | 7.057 ±0.802 | 5.328 ±1.007 | 5.353 ±0.633 |

| | | | |
|---|---|---|---|
| The data represent the mean ± standard deviation from 3 animals | | | |

### REFERENCES

U.S. Patent 4,141,654
U.S. Patent 5,268,163
U.S. Patent 5,648,063
U.S. Patent 5,880,281
U.S. Patent 5,955,053
U.S. Patent 5,986,074
U.S. Patent 6,691,724
U.S. Patent 5,605,672
U.S. Patent 6,071,490
U.S. Patent 6,613,305
U.S. Patent 6,737,247

AU Appln. 01/75210A5
PCT Appln. WO 01/80906A2
PCT Appln. WO 01/91807A2
PCT Appln. WO 91/16076A1

Alauddin and Conti, Nucl. Med. Biol., 25(3):175-180 1998.
Alauddin et al., J. Nucl. Med., 42(11):1682-1690, 2001.
Alauddin et al., Nucl. Med. Biol., 23(6):787-792 1996.
Alauddin et al., Nucl. Med. Biol., 26:371-376, 1999.
Blondeau et al., Can. J. Chem., 45:49-52, 1967.
Connors, Ann. Oncol., 7(5):445-52, 1996.
Davison et al., Inorg. Chem, 20:1629-1632, 1980.
Gambhir et al., J. Nucl. Med., 39(11):2003-2011, 1998.
Gambhir et al., Proc. Natl. Acad. Sci. USA, 96(5):2333-2338, 1999.
Gambhir et al., Proc. Natl. Acad. Sci. USA, 97:2785-2790, 2000.
Goldsmith, Sem. Nucl. Med., 27:85-93, 1997.
Iyer et al., J. Nucl. Med., 42(1):96-105, 2001.
Mathias et al., J. Nucl. Med., 37:1003-1008, 1996.
Mathias et al., J. Nucl. Med., 38:133P, 1997b.
Mathias et al., J. Nucl. Med., 38:87P, 1997a.
Namavari et al., Nucl. Med. Biol., 27(2):157-162 2000.
Reed, Cancer Cell, 3:17-22, 2003.
Remington's Pharmaceutical Sciences, 18th Ed. Mack Printing Company, 1289-1329, 1990.
Seabold et al., J. Nucl. Med., 40(9):1434-1440,1999.
Srivastava et al., J. Tox. Env. Health, 47:173-182, 1996.
Surma et al., Nucl. Med. Comm., 15:628- 635, 1994.
Tjuvajev et al., J. Nucl. Med., 43(8):1072-1083 ,2002.
Van Nerom et al., Eur. J. Nucl. Med., 16:417, 1990.
Van Nerom et al., Eur. J. Nucl. Med., 20:738-746, 1993.
Verbruggen et al., Eur. J. Nucl. Med., 16:429, 1990.
Verbruggen et al., J. Nucl. Med., 33:551-557, 1992.
Yaghoubi et al., J. Nucl. Med., 42:1225-1234, 2001.

## Claims

1. A compound defined as haying the formula: 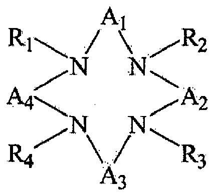 wherein
- A₁, A₂, A₃, and A₄ are each independently -(CH₂)ₓ-, wherein x = 2-4;
- R₁, R₂, and R₃ are each independently hydrogen or 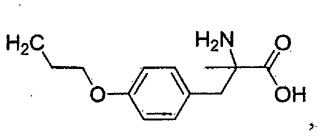 and
- R₄ is

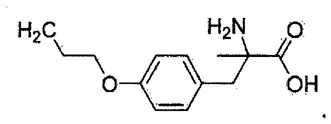

2. The compound of claim 1, wherein the compound is chelated to a metal atom.

3. The compound of claim 2, wherein the metal atom is not radioactive.

4. The compound of claim 3, wherein the metal atom is copper, cobalt, platinum, iron, arsenic, rhenium, or germanium.

5. The compound of claim 1, wherein the compound is chelated to a radionuclide.

6. The compound of claim 5, wherein the radionuclide is ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu, or ⁶²Cu; ⁶⁸Ga, ⁹⁰Y, ⁶⁸Ga, and ¹⁸⁸Re being preferred; and ^{99m}Tc being particularly preferred.

7. A pharmaceutical composition comprising the compound of claim 1.

8. The compound of claim 1 or 6 for use in a method of treating cancer or in an imaging method.

9. The compound for the use of claim 8, wherein the cancer treating method or the imaging method is to be performed in a mammal, in particular in a human.

10. The compound for the use of claim 8, wherein the treatment of cancer is to be performed in combination with a second anti-cancer compound.

11. The compound for the use of claim 8, wherein the treatment of cancer is to be performed in combination with radiation therapy or surgery.

12. The compound for the use of claim 8, wherein imaging is PET or SPET imaging.

13. A kit comprising a compound of claim 1 and a reducing agent.

14. The kit of claim 13, the kit further comprising a radionuclide, in particular a radionuclide such as ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu, or ⁶²Cu.

15. The kit of claim 13, the kit further comprising an antioxidant, in particular an antioxidant such as vitamin C, tocopherol, pyridoxine, thiamine, or rutin.

16. The kit of claim 13, the kit further comprising a transition chelator, in particular a transition chelator such as glucoheptonate, gluconate, glucarate, citrate, or tartarate.

17. The kit of claim 13, wherein the reducing agent is tin (II) chloride or triphenylphosphine.

## Patentansprüche

1. Verbindung definiert mit der Formel: 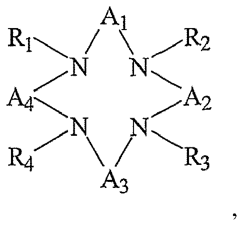 wobei
- A₁, A₂, A₃ und A₄ jeweils unabhängig voneinander -(CH₂)ₓ- sind, wobei x = 2-4;
- R₁, R₂ und R₃ jeweils unabhängig voneinander Wasserstoff oder 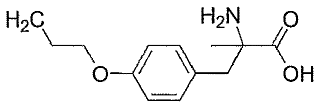 sind, und
- R₄ 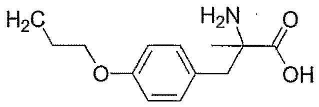
ist.

2. Verbindung nach Anspruch 1, wobei die Verbindung an ein Metallatom chelatiert ist.

3. Verbindung nach Anspruch 2, wobei das Metallatom nicht radioaktiv ist.

4. Verbindung nach Anspruch 3, wobei das Metallatom Kupfer, Kobalt, Platin, Eisen, Arsen, Rhenium oder Germanium ist.

5. Verbindung nach Anspruch 1, wobei die Verbindung an ein Radionuklid chelatiert ist.

6. Verbindung nach Anspruch 5, wobei das Radionuklid ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, 64Cu oder ⁶²Cu ist, wobei ⁶⁸Ga, ⁹⁰Y, ⁶¹Ga und ¹⁸⁸Re bevorzugt sind und ^{99m}Tc besonders bevorzugt ist.

7. Pharmazeutische Zusammensetzung umfassend die Verbindung nach Anspruch 1.

8. Verbindung nach Anspruch 1 oder 6 zur Verwendung in einem Verfahren zur Behandlung von Krebs oder in einem bildgebenden Verfahren.

9. Verbindung zur Verwendung nach Anspruch 8, wobei das Krebs-Behandlungsverfahren oder das bildgebende Verfahren in einem Säugetier, insbesondere in einem Menschen durchgeführt werden soll.

10. Verbindung zur Verwendung nach Anspruch 8, wobei die Behandlung von Krebs in Kombination mit einer zweiten Antikrebs-Verbindung durchgeführt werden soll.

11. Verbindung zur Verwendung nach Anspruch 8, wobei die Behandlung von Krebs in Kombination mit Strahlentherapie oder Operation durchgefühlt werden soll.

12. Verbindung zur Verwendung nach Anspruch 8, wobei die Bildgebung PET- oder SPET-Bildgebung ist.

13. Kit, umfassend eine Verbindung nach Anspruch 1 und ein Reduktionsmittel.

14. Kit nach Anspruch 13, wobei das Kit weiterhin ein Radionuklid umfasst, insbesondere ein Radionuklid wie ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu oder ⁶²Cu.

15. Kit nach Anspruch 13, wobei das Kit weiterhin ein Antioxidans umfasst, insbesondere ein Antioxidans wie Vitamin C, Tocopherol, Pyridoxin, Thiamin oder Rutin.

16. Kit nach Anspruch 13, wobei das Kit weiterhin einen Übergangschelator umfasst, insbesondere einen Übergangschelator wie Glucoheptonat, Gluconat, Glucarat, Citrat oder Tartrat.

17. Kit nach Anspruch 13, wobei das Reduktionsmittel Zinn(II)-Chlorid oder Triphenylphosphin ist.

## Revendications

1. Composé défini comme ayant la formule : 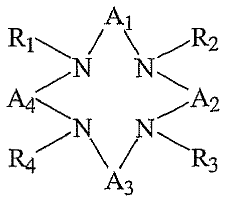 dans laquelle
- A₁, A₂, A₃, et A₄ sont chacun indépendamment -(CH₂)ₓ-, où x = 2-4 ;
- R₁, R₂, et R₃ sont chacun indépendamment un hydrogène ou 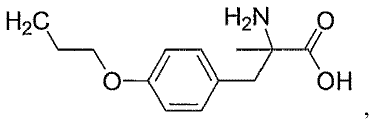 et
- R4 est

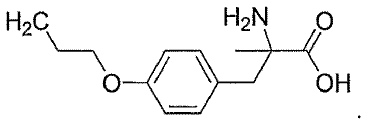

2. Composé selon la revendication 1, dans lequel le composé est chélaté à un atome métallique.

3. Composé selon la revendication 2, dans lequel l'atome métallique n'est pas radioactif.

4. Composé selon la revendication 3, dans lequel l'atome métallique est du cuivre, du cobalt, du platine, du fer, de l'arsenic, du rhénium, ou du germanium.

5. Composé selon la revendication 1, dans lequel le composé est chélaté à un radionucléide.

6. Composé selon la revendication 5, dans lequel le radionucléide est ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu, ou ⁶²Cu ; ⁶⁸Ga, ⁹⁰Y, ⁶⁸Ga, et ¹⁸⁸Re étant préférés ; et ^{99m}Tc étant particulièrement préféré.

7. Composition pharmaceutique comprenant le composé selon la revendication 1.

8. Composé selon la revendication 1 ou 6 pour une utilisation dans une méthode de traitement d'un cancer ou dans une méthode d'imagerie.

9. Composé pour l'utilisation selon la revendication 8, dans lequel la méthode de traitement d'un cancer ou la méthode d'imagerie est destinée à être réalisée chez un mammifère, en particulier chez un humain.

10. Composé pour l'utilisation selon la revendication 8, dans lequel le traitement d'un cancer est destiné à être réalisé en combinaison avec un second composé anti-cancéreux.

11. Composé pour l'utilisation selon la revendication 8, dans lequel le traitement d'un cancer est destiné à être réalisé en combinaison avec une radiothérapie ou une radiochirurgie.

12. Composé pour l'utilisation selon la revendication 8, dans lequel l'imagerie est une imagerie PET ou SPET.

13. Kit comprenant un composé selon la revendication 1 et un agent réducteur.

14. Kit selon la revendication 13, le kit comprenant en outre un radionucléide, en particulier un radionucléide tel que ^{99m}Tc, ¹⁸⁸Re, ¹⁸⁶Re, ¹⁸³Sm, ¹⁶⁶Ho, ⁹⁰Y, ⁸⁹Sr, ⁶⁷Ga, ⁶⁸Ga, ¹¹¹In, ¹⁸³Gd, ⁵⁹Fe, ²²⁵Ac, ²¹²Bi, ²¹¹At, ⁴⁵Ti, ⁶⁰Cu, ⁶¹Cu, ⁶⁷Cu, ⁶⁴Cu ou ⁶²Cu.

15. Kit selon la revendication 13, le kit comprenant en outre un antioxydant, en particulier un antioxydant tel que la vitamine C, le tocophérol, la pyridoxine, la thiamine, ou la rutine.

16. Kit selon la revendication 13, le kit comprenant en outre un chélateur de transition, en particulier un chélateur de transition tel que le glucoheptonate, le gluconate, le glucarate, le citrate, ou le tartrate.

17. Kit selon la revendication 13, dans lequel l'agent réducteur est du chlorure d'étain (II) ou de la triphénylphosphine.
